(19) Europäisches Patentamt
European Patent Office
Office européen des brevets

(11) Veröffentlichungsnummer: **0 591 781 A1**

(12) **EUROPÄISCHE PATENTANMELDUNG**

(21) Anmeldenummer: **93115420.7**

(22) Anmeldetag: **24.09.93**

(51) Int. Cl.5: **C07D 403/04**, C07D 417/04, C07D 413/04, C07D 231/22, A01N 47/38

(30) Priorität: **07.10.92 DE 4233715**

(43) Veröffentlichungstag der Anmeldung: **13.04.94 Patentblatt 94/15**

(84) Benannte Vertragsstaaten: **BE CH DE ES FR GB IT LI NL**

(71) Anmelder: **BAYER AG**
**D-51368 Leverkusen(DE)**

(72) Erfinder: **Fuchs, Rainer, Dr.**
**Am Rohm 107**
**D-42113 Wuppertal(DE)**
Erfinder: **Kanellakopulos, Johannes, Dr.**
**Mozartstrasse 94c**
**D-40724 Hilden(DE)**
Erfinder: **Erdelen, Christoph, Dr.**
**Unterbüscherhof 22**
**D-42799 Leichlingen(DE)**
Erfinder: **Stendel, Wilhelm, Dr.**
**In den Birken 56**
**D-42113 Wuppertal(DE)**

(54) **Substituierte Carbamoylpyrazoline, Verfahren zu ihrer Herstellung und ihre Verwendung als Schädlingsbekämpfungsmittel.**

(57) Die Erfindung betrifft neue substituierte Carbamoylpyrazoline

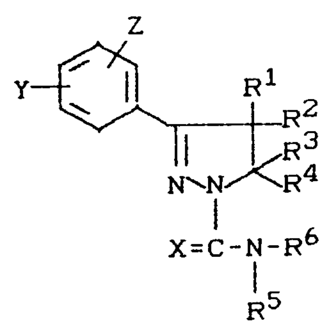

(I)

in welcher

$R^1$ für einen jeweils gegebenenfalls substituierten und über Stickstoff gebundenen Azolinon-, Azolinthion- oder Azoliniminorest steht,

Verfahren in ihrer Herstellung und ihre Verwendung als Schädlingsbekämpfungsmittel.

EP 0 591 781 A1

Die Erfindung betrifft neue substituierte Carbamoylpyrazoline, Verfahren zu ihrer Herstellung und ihre Verwendung als Schädlingsbekämpfungsmittel.

Es ist bekannt, daß bestimmte Pyrazolinderivate eine gute Wirksamkeit gegen tierische Schädlinge besitzen.

Siehe dazu z.B. DE-A 2 700 258, US-A 4 174 393, DE-A 2 529 689, US-A 4 070 365 und EP-A 0 466 408.

Die Wirkungshöhe bzw. Wirkungsdauer dieser vorbekannten Verbindungen ist jedoch, insbesondere bei bestimmten Organismen oder bei niedrigen Anwendungskonzentrationen nicht in allen Anwendungsgebieten zufriedenstellend.

Es wurden neue substituierte Carbamoylpyrazoline der allgemeinen Formel(I),

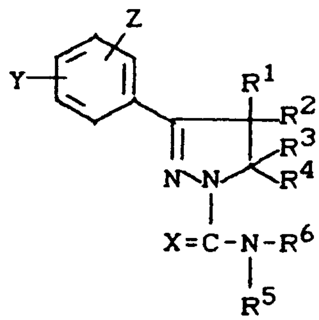

(I)

in welcher

$R^1$ für einen jeweils gegebenenfalls substituierten und über Stickstoff gebundenen Azolinon-, Azolinthion- oder Azoliniminorest steht,

$R^2$ für Wasserstoff, Alkyl, gegebenenfalls substituiertes Cycloalkyl, Halogenalkyl, Halogenalkylthio oder Alkoxycarbonyl steht,

$R^3$ für Wasserstoff, Alkyl oder für eine Gruppierung

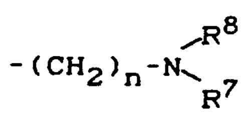

oder $-(CH_2)_n-O-R^9$ steht,

worin

$R^7$ und $R^8$ jeweils unabhängig voneinander für Wasserstoff, Alkyl oder Aryl stehen und

$R^9$ für Wasserstoff, Alkyl oder Aryl steht und

n für die Zahlen 1 bis 6 steht,

$R^4$ für Wasserstoff oder Alkyl steht,

$R^5$ für Wasserstoff, Alkyl, Phenyl oder Alkylthio steht,

$R^6$ für gegebenenfalls substituiertes Alkyl, gegebenenfalls substituiertes Cycloalkyl oder für den Rest

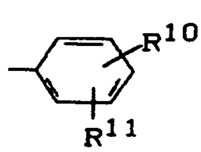

steht,

worin $R^{10}$ und $R^{11}$ gleich oder verschieden sein können und für Wasserstoff, Halogen, Alkyl, Nitro, Cyano, Halogenalkyl, Alkoxy, Halogenalkoxy, Alkylthio, Halogenalkylthio, gegebenenfalls substituiertes Phenoxy, gegebenenfalls substituiertes Mono- oder Dialkylamino, gegebenenfalls substituiertes Cycloalkyl, Alkoxycarbonyl, gegebenenfalls substituiertes

Arylthio, Alkenyloxy, Alkinyl, Alkylthionyl, Alkylsulfonyl, Halogenalkylthionyl, Halogenalkylsulfonyl, Halogenalkoxycarbonyl stehen oder wobei $R^{10}$ und $R^{11}$ zusammen für einen bivalenten gegebenenfalls ein oder zwei Sauerstoffatome enthaltenden und gegebenenfalls substituierten Rest stehen,

X für Sauerstoff oder Schwefel steht und

Y und Z gleich oder verschieden sein können und für Wasserstoff, Alkyl, Halogen, Halogenalkyl, Alkoxy, Alkylthio, Halogenalkoxy, Halogenalkylthio, Alkoxycarbonyl, Halogenalkoxycarbonyl, gegebenenfalls substituiertes Aryloxy, gegbenenfalls substituiertes Arylthio, Alkenyloxy, Alkinyl, Alkylthionyl, Alkylsulfonyl, Halogenalkylthionyl, Halogenalkylsulfonyl, Nitro oder Cyano stehen oder wobei Y und Z zusammen für gegebenenfalls durch Halogen substituiertes 3,4-Methylendioxy oder 3,4-Ethylendioxy stehen,

gefunden.

Weiterhin wurde gefunden, daß man die neuen substituierten Carbamoylpyrazoline der allgemeinen Formel (I)

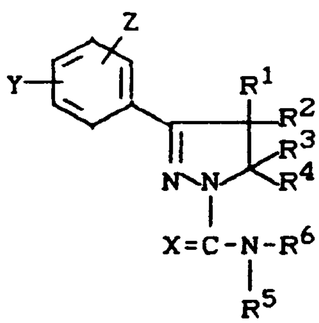

(I)

in welcher

$R^1$ für einen jeweils gegebenenfalls substituierten Azolinon-, Azolinthion-oder Azoliniminorest steht,

$R^2$ für Wasserstoff, Alkyl, gegebenenfalls substituiertes Cycloalkyl, Halogenalkyl, Halogenalkylthio oder Alkoxycarbonyl steht,

$R^3$ für Wasserstoff, Alkyl oder für eine Gruppierung

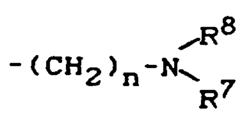

oder $-(CH_2)_n-O-R^9$ steht,

worin

$R^7$ und $R^8$ jeweils unabhängig voneinander für Wasserstoff, Alkyl oder Aryl stehen und

$R^9$ für Wasserstoff, Alkyl oder Aryl steht und

n für die Zahlen 1 bis 6 steht,

$R^4$ für Wasserstoff oder Alkyl steht,

$R^5$ für Wasserstoff, Alkyl, Phenyl oder Alkylthio steht,

$R^6$ für gegebenenfalls substituiertes Alkyl, gegebenenfalls substituiertes Cycloalkyl oder für den Rest

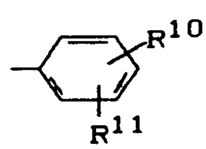

steht,

worin $R^{10}$ und $R^{11}$ gleich oder verschieden sein können und für Wasserstoff, Halogen, Alkyl, Nitro, Cyano, Halogenalkyl, Alkoxy, Halogenalkoxy, Alkylthio, Halogenalkylthio, gegebenenfalls substituiertes Phenoxy, gegebenenfalls substituiertes Mono- oder Dialkylamino, gegebenenfalls substituiertes Cycloalkyl, Alkoxycarbonyl, gegebenenfalls substituiertes Arylthio, Alkenyloxy, Alkinyl, Alkylthionyl, Alkylsulfonyl, Halogenalkylthionyl, Halogenalkylsulfonyl, Halogenalkoxycarbonyl stehen oder wobei $R^{10}$ und $R^{11}$ zusammen für einen bivalenten gegebenenfalls ein oder zwei Sauerstoffatome enthaltenden und gegebenenfalls substituierten Rest stehen,

X für Sauerstoff oder Schwefel steht und

Y und Z gleich oder verschieden sein können und für Wasserstoff, Alkyl, Halogen, Halogenalkyl, Alkoxy, Alkylthio, Halogenalkoxy, Halogenalkylthio, Alkoxycarbonyl, Halogenalkoxycarbonyl, gegebenenfalls substituiertes Aryloxy, gegbenenfalls substituiertes Arylthio, Alkenyloxy, Alkinyl, Alkylthionyl, Alkylsulfonyl, Halogenalkylthionyl, Halogenalkylsulfonyl, Nitro oder Cyano stehen oder wobei Y und Z zusammen für gegebenenfalls durch Halogen substituiertes 3,4-Methylendioxy oder 3,4-Ethylendioxy stehen,

erhält, wenn man

A) zum Erhalt von Carbamoylpyrazolinen der Formel (I), in welcher $R^5$ für Wasserstoff steht, Pyrazolinderivate der Formel (II)

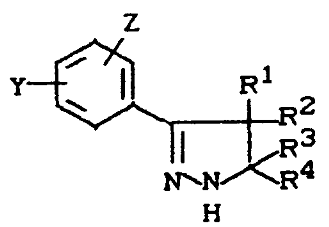

(II)

in welcher Y, Z, $R^1$, $R^2$, $R^3$ und $R^4$ die oben angegebene Bedeutung besitzen,
mit Isocyanaten bzw. Isothiocyanaten der Formel (III)

$X=C=N\text{-}R^6$ (III)

in welcher X und $R^6$ die oben angegebene Bedeutung haben,
gegebenenfalls in Gegenwart von Basen umsetzt
oder wenn man

B) zum Erhalt von Carbamoylpyrazolinen der Formel (I), in welcher $R^2$ für Alkyl, Cycloalkyl, Halogenalkyl, Halogenalkylthio oder Alkoxycarbonyl steht, Verbindungen der Formel (IV)

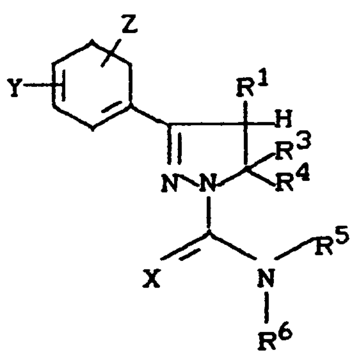

(IV)

in welcher X, Y, Z, $R^1$, $R^3$, $R^4$, $R^5$ und $R^6$ die oben angegebene Bedeutung haben mit Verbindungen der Formel (V)

$Hal\text{-}R^{2-1}$ (V)

in welcher

Hal für Halogen steht und

$R^{2-1}$ für Alkyl, gegebenenfalls substituiertes Cycloalkyl, Halogenalkyl, Halogenalkylthio oder Alkoxycarbonyl steht,

in wasserfreiem Medium unter Zusatz einer starken Base umsetzt.

Schließlich wurde gefunden, daß die neuen Carbamoylpyrazoline der allgemeinen Formel (I) eine sehr gute Wirkung gegen Schädlinge und insbesondere eine sehr gute insektizide und akarizide Wirksamkeit besitzen.

Überraschenderweise zeigen die erfindungsgemäßen substituierten Carbamoylpyrazoline eine erheblich bessere insektizide Wirksamkeit gegen pflanzenschädigende und warmblüterparasitierende Insekten und Spinnentiere als aus dem Stand der Technik bekannte chemisch und wirkungsmäßig naheliegende Verbindungen.

Die erfindungsgemäßen substituierten Carbamoylpyrazoline sind durch die Formel (I) allgemein definiert. Bevorzugt sind Verbindungen der Formel (I), bei welchen

$R^1$ für einen gegebenenfalls einfach oder zweifach, gleich oder verschieden substituierten und über Stickstoff gebundenen Azolinon-, Azolinthion- oder Azoliniminorest aus der Reihe

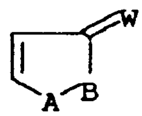
($R^1$-a)

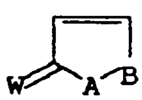
($R^1$-b)

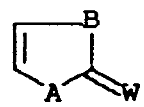
($R^1$-c)

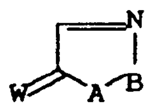
($R^1$-d)

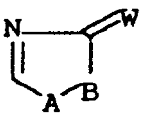
($R^1$-e)

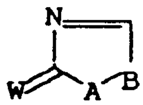
($R^1$-f)

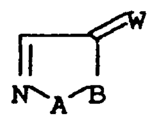
($R^1$-g)

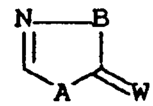
($R^1$-h)

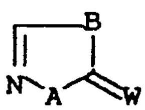
($R^1$-i)

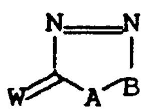
($R^1$-k)

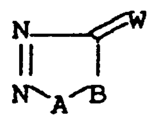
($R^1$-l)

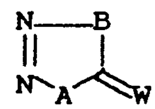
($R^1$-m)

steht

worin

eine der Gruppen A oder B für Stickstoff steht und jeweils die andere (B oder A) für Sauerstoff, Schwefel oder für die Gruppe -N Alkyl($C_1$-$C_4$) oder für eine Methylengruppierung -$CH_2$- steht,

W für Sauerstoff, Schwefel oder für die Gruppe -N Alkyl($C_1$-$C_4$) steht,

und wobei folgende Substituenten infrage kommen: Alkyl ($C_1$-$C_6$), Alkoxy($C_1$-$C_6$), Alkyl($C_1$-$C_6$)thio, Amino, Alkyl ($C_1$-$C_6$)amino, Halogenalkyl($C_1$-$C_4$), Dialkyl($C_1$-$C_6$)amino und gegebenenfalls durch Halogen, Alkyl($C_1$-$C_4$), Alkoxy ($C_1$-$C_4$), Halogenalkyl($C_1$-$C_4$), Halogenalkoxy($C_1$-$C_4$)- oder Halogenalkyl($C_1$-$C_4$)thio substituiertes Phenyl,

$R^2$ für Wasserstoff, Alkyl($C_1$-$C_6$), gegebenenfalls durch Halogen, Halogenalkyl($C_1$-$C_4$) substituiertes Cycloalkyl($C_3$-$C_7$); Halogenalkyl($C_1$-$C_4$), Halogenalkyl($C_1$-$C_4$)thio oder Alkoxy($C_1$-$C_6$)carbonyl steht,

$R^3$ für Wasserstoff, Alkyl($C_1$-$C_6$) oder für eine Gruppierung

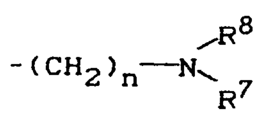

oder $-(CH_2)_n—O-R^9$ steht
worin

$R^7$ und $R^8$ jeweils unabhängig voneinander für Wasserstoff, Alkyl($C_1$-$C_6$) oder für gegebenenfalls durch Alkyl($C_1$-$C_4$), Alkoxy($C_1$-$C_4$), Alkyl($C_1$-$C_4$)thio, Halogen, Halogenalkyl($C_1$-$C_4$), Halogenalkoxy($C_1$-$C_4$) oder Halogenalkyl($C_1$-$C_4$)thio substituiertes Phenyl stehen und

$R^9$ für Wasserstoff, Alkyl($C_1$-$C_6$) oder gegebenenfalls substituiertes Phenyl steht, wobei als Substituenten die für $R^8$ aufgeführten Phenylsubstituenten infrage kommen und

n für die Zahlen 1 bis 4 steht,

$R^4$ für Wasserstoff oder Alkyl($C_1$-$C_6$) steht,

$R^5$ für Wasserstoff, Alkyl($C_1$-$C_6$), Phenyl oder Alkyl($C_1$-$C_6$)thio steht,

$R^6$ für gegebenenfalls durch Halogen, Halogenalkyl($C_1$-$C_4$), Halogenalkoxy($C_1$-$C_4$) substituiertes Alkyl($C_1$-$C_6$), für gegebenenfalls durch Halogen, Halogenalkyl($C_1$-$C_4$),Halogenalkoxy-($C_1$-$C_4$) substituiertes Cycloalkyl($C_3$-$C_7$) oder für den Rest

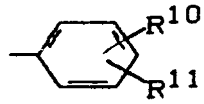

steht,

wobei

$R^{10}$ und $R^{11}$ gleich oder verschieden sein können und für Halogen, Alkyl($C_1$-$C_6$), Nitro, Cyano, Halogenalkyl($C_1$-$C_4$), Alkoxy($C_1$-$C_6$), Halogenalkoxy($C_1$-$C_4$), Alkyl($C_1$-$C_4$)thio, Halogenalkyl($C_1$-$C_4$)thio, gegebenenfalls durch Halogen, Halogenalkyl($C_1$-$C_4$), Alkoxy($C_1$-$C_4$) substituiertes Phenoxy oder Phenylthio, gegebenenfalls durch Halogen, Alkoxy($C_1$-$C_4$), Halogenalkyl($C_1$-$C_4$) substituiertes Mono- oder Dialkylamino mit jeweils 1 bis 6 Kohlenstoffatomen im Alkylrest, gegebenenfalls durch Alkyl($C_1$-$C_4$), Alkoxy($C_1$-$C_4$), Halogen, Alkyl($C_1$-$C_4$)thio substituiertes Cycloalkyl($C_3$-$C_7$), Alkoxy($C_1$-$C_4$)carbonyl, Alkenyl($C_2$-$C_6$)oxy, Alkinyl($C_2$-$C_6$), Alkyl($C_1$-$C_4$)thionyl, Alkyl($C_1$-$C_4$)sulfonyl, Halogenalkyl($C_1$-$C_4$)thionyl, Halogenalkyl($C_1$-$C_4$)sulfonyl, Halogenalkoxy($C_1$-$C_4$)carbonyl stehen oder wobei

$R^{10}$ und $R^{11}$ zusammen für einen der folgenden bivalenten Reste stehen

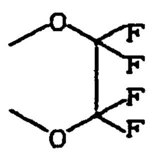 , 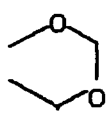 , 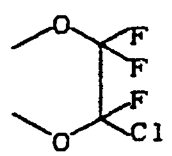 , 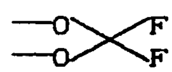 ,

X für Sauerstoff oder Schwefel steht und

Y und Z gleich oder verschieden sein können und für Wasserstoff, Alkyl($C_1$-$C_6$), Halogen, Halogenalkyl($C_1$-$C_6$), Alkoxy($C_1$-$C_6$), Alkyl($C_1$-$C_6$)thio, Halogenalkoxy($C_1$-$C_4$), Halogenalkyl($C_1$-$C_4$)thio, Alkoxy($C_1$-$C_4$)carbonyl, Halogenalkoxy($C_1$-$C_4$)carbonyl, gegebenenfalls durch Halogen, Alkyl($C_1$-$C_4$), Alkoxy($C_1$-$C_4$), Halogenalkyl ($C_1$-$C_4$) substituiertes Phenoxy oder Phenylthio, Alkenyl($C_2$-$C_6$)oxy, Alkinyl($C_2$-$C_6$), Alkyl($C_1$-$C_4$)thionyl, Alkyl-($C_1$-$C_4$)sulfonyl, Halogenalkyl($C_1$-$C_4$)thionyl, Halogenalkyl($C_1$-$C_4$)sulfonyl, Nitro, Cyano stehen oder wobei

Y und Z zusammen für gegebenenfalls durch Fluor und/oder Chlor substituiertes 3,4-Methylendioxy oder 3,4-Ethylendioxy stehen.

Besonders bevorzugt sind Verbindungen der Formel (I), bei welchen

$R^1$ für einen gegebenenfalls einfach oder zweifach, gleich oder verschieden substituierten und über Stickstoff gebundenen Azolinon-, Azolinthion- oder Azoliniminorest aus der Reihe

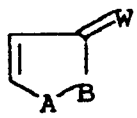
($R^1$-a)

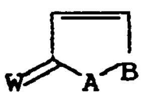
($R^1$-b)

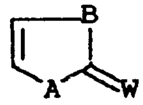
($R^1$-c)

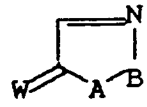
($R^1$-d)

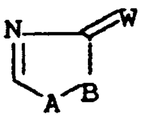
($R^1$-e)

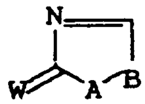
($R^1$-f)

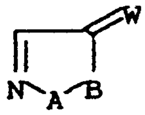
($R^1$-g)

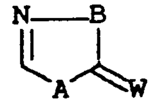
($R^1$-h)

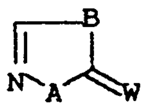
($R^1$-i)

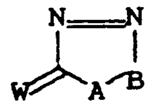
($R^1$-k)

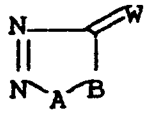
($R^1$-l)

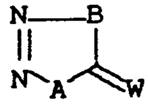
($R^1$-m)

steht,

worin
eine der Gruppen A oder B für Stickstoff steht und jeweils die andere (B oder A) für Sauerstoff, Schwefel oder für die Gruppe -N Alkyl($C_1$-$C_4$) oder für eine Methylengruppierung -$CH_2$- steht,
W für Sauerstoff, Schwefel oder für die Gruppe -N Alkyl ($C_1$-$C_4$) steht,
und wobei folgende Substituenten infrage kommen: Alkyl ($C_1$-$C_4$), Alkoxy($C_1$-$C_4$), Alkyl($C_1$-$C_4$)thio, Amino, Alkyl ($C_1$-$C_4$)amino Halogenalkyl($C_1$-$C_4$), Dialkyl($C_1$-$C_4$)amino und gegebenenfalls durch Fluor, Chlor, Brom, Methyl, Ethyl, Methoxy, Ethoxy, Halogenalkyl($C_1$-$C_2$), Halogenalkoxy($C_1$-$C_2$) oder Halogenalkyl($C_1$-$C_2$)thio substituiertes Phenyl,

$R^2$ für Wasserstoff, Alkyl($C_1$-$C_4$), gegebenenfalls durch Fluor, Chlor, Brom, Halogenalkyl($C_1$-$C_3$) substituiertes Cycloalkyl($C_3$-$C_6$); Halogenalkyl($C_1$-$C_3$), Halogenalkyl($C_1$-$C_3$)thio oder Alkoxy($C_1$-$C_4$)carbonyl steht,

$R^3$ für Wasserstoff, Alkyl($C_1$-$C_4$) oder für eine Gruppierung

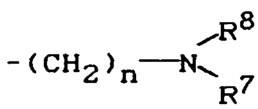

oder -$(CH_2)_n$—O-$R^9$ steht
worin

$R^7$ und $R^8$ jeweils unabhängig voneinander für Wasserstoff, Alkyl($C_1$-$C_4$) oder für gegebenenfalls durch Methyl, Ethyl, Methoxy, Ethoxy, Methylthio, Ethylthio, Fluor, Chlor, Brom, Halogenalkyl($C_1$-$C_2$), Halogenalkoxy($C_1$-$C_2$) oder Halogenalkyl($C_1$-$C_2$)thio substituiertes Phenyl stehen und

$R^9$ für Wasserstoff, Alkyl($C_1$-$C_4$) oder gegebenenfalls einfach oder mehrfach, gleich oder verschieden substituiertes Phenyl steht, wobei als Substituenten die für $R^8$ aufgeführten Phenylsubstituenten infrage kommen und

n für die Zahlen 1, 2 oder 3 steht,

$R^4$ für Wasserstoff oder Alkyl($C_1$-$C_4$) steht,

$R^5$ für Wasserstoff, Alkyl($C_1$-$C_4$), Phenyl oder Alkyl($C_1$-$C_3$)thio steht

$R^6$ für gegebenenfalls durch Fluor, Chlor, Brom, Halogenalkyl($C_1$-$C_3$), Halogenalkoxy($C_1$-$C_3$) substituiertes Alkyl($C_1$-$C_4$), für gegebenenfalls durch Fluor, Chlor, Brom, Halogenalkyl($C_1$-$C_3$), Halogenalkoxy($C_1$-$C_3$) substituiertes Cycloalkyl($C_3$-$C_6$) oder für den Rest

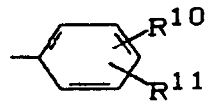

steht,

wobei

$R^{10}$ und $R^{11}$ gleich oder verschieden sein können und für Fluor, Chlor, Brom, Jod, Alkyl($C_1$-$C_4$), Nitro, Cyano, Halogenalkyl($C_1$-$C_3$), Alkoxy($C_1$-$C_4$), Halogenalkoxy($C_1$-$C_3$), Alkyl($C_1$-$C_3$)thio, Halogenalkyl($C_1$-$C_3$)thio, gegebenenfalls durch Fluor, Chlor, Brom, Halogenalkyl($C_1$-$C_3$), Alkoxy($C_1$-$C_4$) substituiertes Phenoxy, gegebenenfalls durch Fluor, Chlor, Brom, Alkoxy-($C_1$-$C_3$), Halogenalkyl($C_1$-$C_3$) substituiertes Mono- oder Dialkylamino mit jeweils 1 bis 4 Kohlenstoffatomen im Alkylrest, gegebenenfalls durch Alkyl($C_1$-$C_3$), Alkoxy($C_1$-$C_3$), Fluor, Chlor, Brom, Alkyl($C_1$-$C_3$)thio substituiertes Cycloalkyl($C_3$-$C_6$), stehen oder wobei

$R^{10}$ und $R^{11}$ zusammen für einen der folgenden bivalenten Reste stehen:

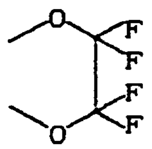 , 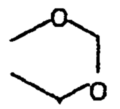 , 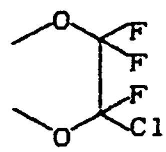 , 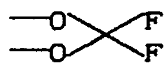 ,

X für Sauerstoff oder Schwefel steht und Y und Z gleich oder verschieden sein können und für Wasserstoff, Alkyl($C_1$-$C_4$), Fluor, Chlor, Brom, Halogenalkyl($C_1$-$C_4$), Alkoxy($C_1$-$C_4$), Alkyl($C_1$-$C_4$)thio, Halogenalkoxy($C_1$-$C_3$), Halogenalkyl($C_1$-$C_3$)thio, Alkoxy($C_1$-$C_3$)-carbonyl, gegebenenfalls durch Fluor, Chlor, Brom, Alkyl($C_1$-$C_3$), Alkoxy($C_1$-$C_3$), Halogenalkyl ($C_1$-$C_3$) substituiertes Phenoxy oder Phenylthio, Alkenyl($C_2$-$C_4$)oxy, Alkinyl($C_2$-$C_4$), Alkyl($C_1$-$C_3$)thionyl, Alkyl($C_1$-$C_3$)sulfonyl, Halogenalkyl($C_1$-$C_3$)thionyl, Halogenalkyl($C_1$-$C_3$)sulfonyl, Nitro, Cyano stehen oder wobei

Y und Z zusammen für gegebenenfalls durch Fluor und/oder Chlor substituiertes 3,4-Methylendioxy oder 3,4-Ethylendioxy stehen.

Insbesondere bevorzugt sind Verbindungen der Formel (I), bei welchen

$R^1$ für einen gegebenenfalls einfach oder zweifach, gleich oder verschieden substituierten und über Stickstoff gebundenen Azolinon-, Azolinthion- oder Azoliniminorest aus der Reihe

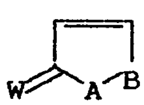

($R^1$-b)

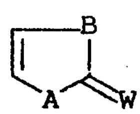

($R^1$-c)

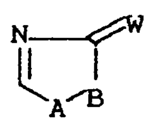

($R^1$-e)

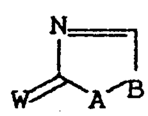

($R^1$-f)

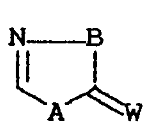

($R^1$-h)

oder

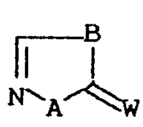

($R^1$-i)

steht ,

worin

eine der Gruppen A oder B für Stickstoff steht und die andere (B oder A) für Sauerstoff, Schwefel oder für die Gruppe -$NCH_3$ oder für eine Methylengruppe -$CH_2$-steht,

W für Sauerstoff oder Schwefel steht,

und wobei folgende Substituenten infrage kommen: Methyl, Ethyl, n-Propyl, i-Propyl, t-Butyl, Methoxy, Methylthio, Trifluormethyl, Chlordifluormethyl, Fluordichlormethyl und gegebenenfalls durch Fluor, Chlor,

Brom, Methyl, Methoxy, Trifluormethyl, Trifluormethoxy substituiertes Phenyl

$R^2$ für Wasserstoff, Methyl, Ethyl, n-Propyl, i-Propyl oder Alkoxy($C_1$-$C_2$)carbonyl steht,

$R^3$ für Wasserstoff, Methyl, Ethyl, n-Propyl, i-Propyl oder für die Gruppe

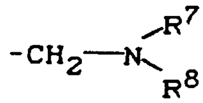

steht,

worin

$R^7$ und $R^8$ jeweils unabhängig voneinander für Wasserstoff, Methyl, Ethyl, n-Propyl, i-Propyl steht,

$R^4$ für Wasserstoff, Methyl, Ethyl oder n-Propyl steht,

$R^5$ für Wasserstoff, Methyl, Ethyl, n-Propyl, i-Propyl, Phenyl oder Alkyl($C_1$-$C_2$)thio steht,

$R^6$ für gegebenenfalls durch Fluor, Chlor, Halogenalkyl($C_1$-$C_3$), Halogenalkoxy($C_1$-$C_3$) substituiertes Methyl, Ethyl, n-Propyl oder i-Propyl, für gegebenenfalls durch Fluor, Chlor, Halogenalkyl($C_1$-$C_3$), Halogenalkoxy($C_1$-$C_3$) substituiertes Cycloalkyl($C_3$-$C_6$) oder für den Rest

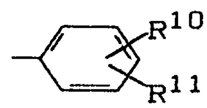

steht,

wobei

$R^{10}$ und $R^{11}$ gleich oder verschieden sein können und für Fluor, Chlor, Brom, Iod, Methyl, Ethyl, n-Propyl, i-Propyl, tert.-Butyl, Nitro, Cyano, Halogenalkyl($C_1$-$C_3$), Alkoxy($C_1$-$C_3$), Halogenalkoxy($C_1$-$C_3$), Alkyl($C_1$-$C_3$)thio, Halogenalkyl($C_1$-$C_3$)thio, gegebenenfalls durch Fluor, Chlor, Halogenalkyl($C_1$-$C_3$), Methoxy, Ethoxy, Methyl, Ethyl substituiertes Phenoxy, gegebenenfalls durch Fluor, Chlor, Methoxy, Ethoxy, Halogenalkyl($C_1$-$C_3$) substituiertes Mono- oder Dialkylamino mit jeweils 1 bis 3 Kohlenstoffatomen im Alkylrest, gegebenenfalls durch Methyl, Ethyl, Methoxy, Ethoxy, Fluor, Chlor, Alkyl($C_1$-$C_3$)thio substituiertes Cycloalkyl($C_3$-$C_6$) stehen oder wobei

$R^{10}$ und $R^{11}$ zusammen für einen der folgenden bivalenten Reste stehen

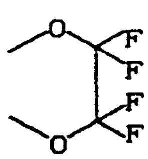 , 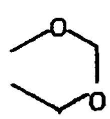 , 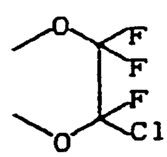 , 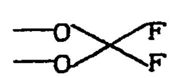 ,

X für Sauerstoff oder Schwefel steht und

Y und Z gleich oder verschieden sein können und für Wasserstoff, Methyl, Ethyl, n-Propyl, i-Propyl, Fluor, Chlor, Halogenalkyl($C_1$-$C_3$), Methoxy, Ethoxy; n-Propyloxy, i-Propyloxy, Alkyl($C_1$-$C_3$)thio, Halogenalkoxy($C_1$-$C_3$), Halogenalkyl($C_1$-$C_3$)thio, Alkoxy($C_1$-$C_3$)carbonyl, gegebenenfalls durch Fluor, Chlor, Methyl, Ethyl, Methoxy, Ethoxy, Halogenalkyl($C_1$-$C_3$) substituiertes Phenoxy oder Phenylthio, Alkenyl($C_3$-$C_4$)oxy, Alkinyl-($C_2$-$C_4$), Alkyl($C_1$-C3)thionyl, Alkyl($C_1$-$C_3$)sulfonyl, Halogenalkyl($C_1$-$C_3$)thionyl, Halogenalkyl($C_1$-$C_3$)sulfonyl, Nitro, Cyano stehen oder wobei

Y und Z zusammen für gegebenenfalls durch Fluor und/oder Chlor substituiertes 3,4-Methylendioxy oder 3,4-Ethylendioxy stehen.

Die Substituentenbedeutung Halogenalkyl in den Resten Halogenalkyl, Halogenalkoxy, Halogenalkylthio, Halogenalkylthionyl und Halogenalkylsulfonyl enthält vorzugsweise 1 bis 4, insbesondere 1 oder 2 Kohlenstoffatome und vorzugsweise 1 bis 5, insbesondere 1 bis 3 gleiche oder verschiedene Halogenatome, wobei als Halogenatome vorzugsweise Fluor, Chlor und Brom, insbesondere Fluor und Chlor stehen. Beispielhaft seien Trifluormethyl, Chlordifluormethyl, Brommethyl, 2,2,2-Trifluorethyl und Pentafluorethyl genannt.

Im einzelnen seien außer den bei den Herstellungsbeispielen genannten Verbindungen die folgenden in den Tabellen 1A bis 1N aufgeführten substituierten Carbamoylpyrazoline der folgenden Formeln genannt:

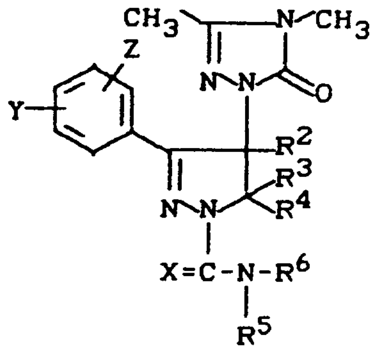

**Tabelle 1A:**

| Y | Z | X | $R^2$ | $R^3$ | $R^4$ | $R^5$ | $R^6$ |
|---|---|---|---|---|---|---|---|
| H | H | O | H | H | H | H | 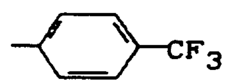 |
| H | H | O | H | H | H | H | 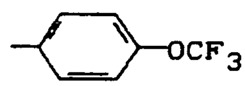 |

| Y | Z | X | $R^2$ | $R^3$ | $R^4$ | $R^5$ | $R^6$ |
|---|---|---|---|---|---|---|---|
| $4-CF_3$ | H | O | H | H | H | H | 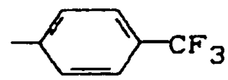 |
| $4-CF_3$ | H | O | H | H | H | H | 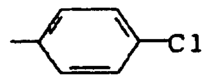 |
| $4-CF_3$ | H | O | H | H | H | H | 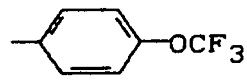 |
| 4-Br | H | O | H | H | H | H | 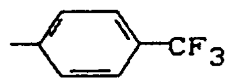 |
| $3-CF_3$ | H | O | H | H | H | H | 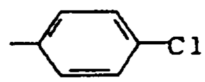 |
| $3-CF_3$ | H | O | H | H | H | H | 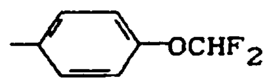 |
| 4-F | H | O | H | H | H | H | 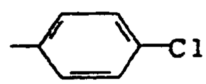 |
| 4-F | H | O | H | H | H | H | 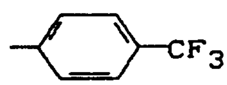 |
| 4-F | H | O | H | H | H | H | 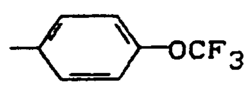 |
| 4-F | H | O | H | H | H | H | 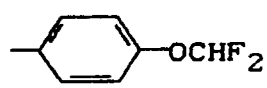 |
| 4-F | H | O | H | H | H | H | 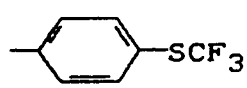 |

| Y | Z | X | $R^2$ | $R^3$ | $R^4$ | $R^5$ | $R^6$ |
|---|---|---|---|---|---|---|---|
| 4-F | H | O | H | H | H | H | 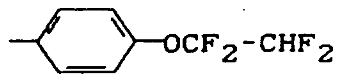 |
| 4-F | H | O | H | H | H | H | 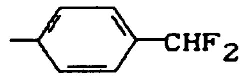 |
| 4-F | H | O | H | H | H | H | 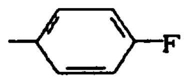 |
| 4-Cl | H | O | H | H | H | H | 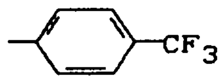 |
| 4-$OCHF_2$ | H | O | H | H | H | H | 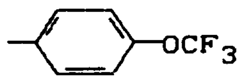 |
| 4-F | H | O | H | H | H | H | 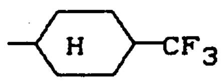 |
| 4-F | H | O | H | H | H | H | 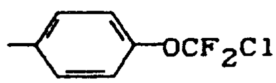 |
| 4-F | H | O | $CH_3$ | H | H | H | 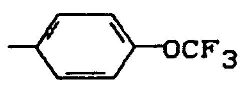 |
| 4-F | H | O | $-COOCH_3$ | H | H | H | 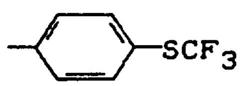 |
| 4-F | H | S | H | H | H | H | 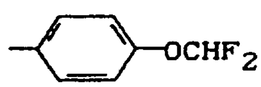 |
| 4-Cl | H | O | H | H | H | H | 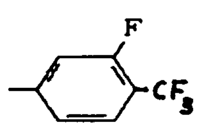 |

| Y | Z | X | $R^2$ | $R^3$ | $R^4$ | $R^5$ | $R^6$ |
|---|---|---|---|---|---|---|---|
| 4-Cl | H | O | H | H | H | H | 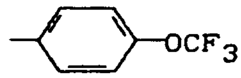 |
| 4-Cl | H | O | H | H | H | H | 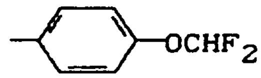 |
| 4-Cl | H | O | H | H | H | H | 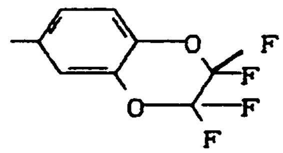 |
| 4-$OCHF_2$ | H | O | H | H | H | H | 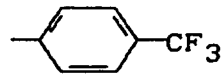 |
| 4-$OCHF_2$ | H | O | H | H | H | H | 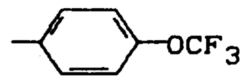 |
| 4-$OCHF_2$ | H | O | H | H | H | H | 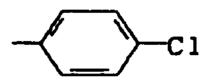 |
| 4-$OCHF_2$ | H | O | H | H | H | H | 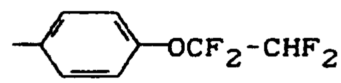 |
| 4-$OCH_2CF_3$ | H | O | H | H | H | H | 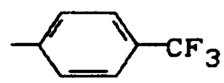 |
| 4-$OCH_2CF_3$ | H | O | H | H | H | H | 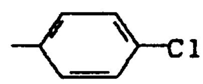 |
| 4-$CF_3$ | H | O | H | H | H | H | 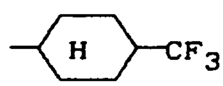 |

Tabelle 1B:

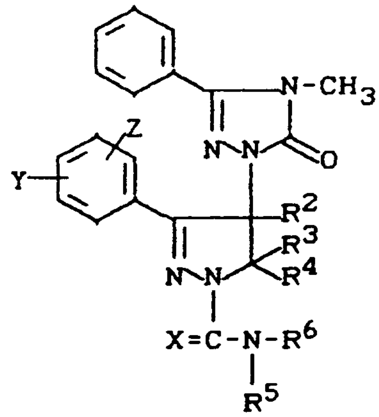

| Y | Z | X | R2 | $R^3$ | $R^4$ | $R^5$ | $R^6$ |
|---|---|---|---|---|---|---|---|
| 4-$CF_3$ | H | O | H | H | H | H | 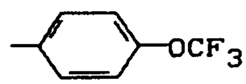 |
| H | H | O | H | H | H | H | 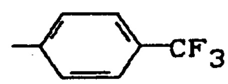 |
| 4-t.-Butyl | H | O | H | H | H | H | 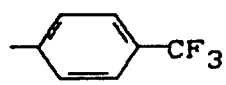 |
| 3-$CF_3$ | H | O | H | H | H | H | 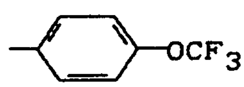 |
| 4-$CH_3$ | H | O | H | H | H | H | 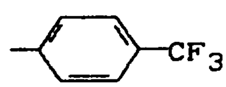 |
| 4-$CH_3$ | H | O | H | H | H | H | 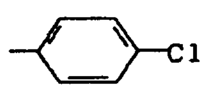 |
| 4-Cl | H | O | H | H | H | H | 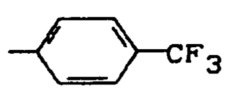 |

| Y | Z | X | $R^2$ | $R^3$ | $R^4$ | $R^5$ | $R^6$ |
|---|---|---|---|---|---|---|---|
| 4-Cl | H | O | H | H | H | H | 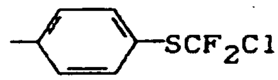 |
| 4-F | H | O | H | H | H | H | 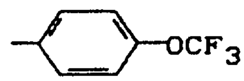 |
| 4-Cl | H | O | H | $CH_3$ | H | H | 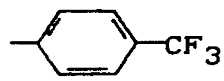 |
| 4-$OCHF_2$ | H | O | H | $CH_3$ | H | H | 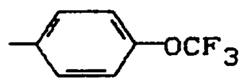 |
| 4-F | H | O | H | $CH_3$ | $CH_3$ | H | 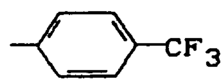 |
| 4-$CF_3$ | H | O | H | H | H | H | 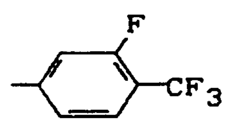 |
| 4-Cl | H | O | H | H | H | H | 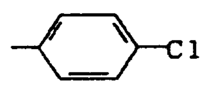 |
| 4-Br | H | O | H | H | H | H | 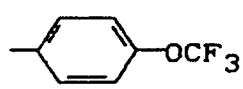 |
| 4-Cl | H | O | H | H | H | H | 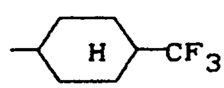 |

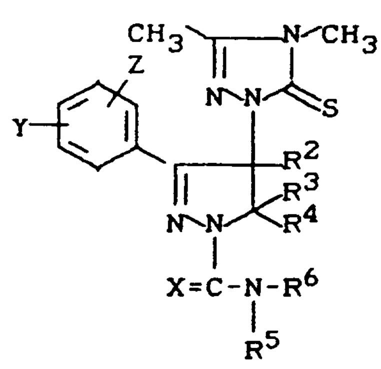

Tabelle 1C:

| Y | Z | X | $R^2$ | $R^3$ | $R^4$ | $R^5$ | $R^6$ |
|---|---|---|---|---|---|---|---|
| H | H | O | H | H | H | H | 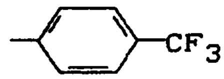 |
| H | H | O | H | H | H | H | 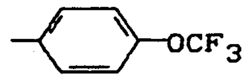 |
| 4-$CF_3$ | H | O | H | H | H | H | 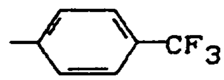 |
| 4-$CF_3$ | H | O | H | H | H | H | 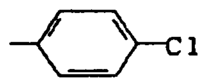 |
| 4-$CF_3$ | H | O | H | H | H | H | 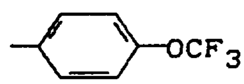 |
| 4-Br | H | O | H | H | H | H | 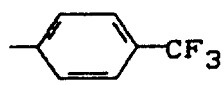 |
| 3-$CF_3$ | H | O | H | H | H | H | 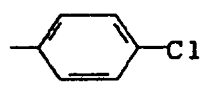 |
| 3-$CF_3$ | H | O | H | H | H | H | 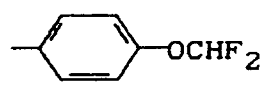 |

| Y | Z | X | $R^2$ | $R^3$ | $R^4$ | $R^5$ | $R^6$ |
|---|---|---|---|---|---|---|---|
| 4-Br | H | O | H | H | H | H | 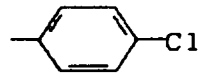 |
| 4-F | H | O | H | H | H | H | 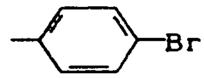 |
| 4-F | H | O | H | H | H | H | 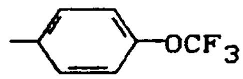 |
| 4-F | H | O | H | H | H | H | 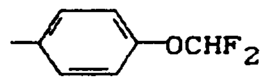 |
| 4-F | H | O | H | H | H | H | 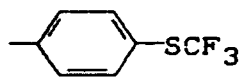 |
| 4-F | H | O | H | H | H | H | 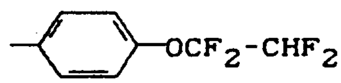 |
| 4-F | H | O | H | H | H | H | 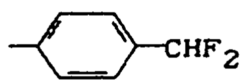 |
| 4-F | H | O | H | H | H | H | 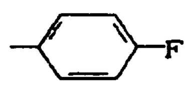 |
| 4-Cl | H | O | H | H | H | H | 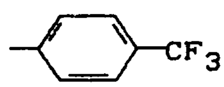 |
| 4-$OCHF_2$ | H | O | H | H | H | H | 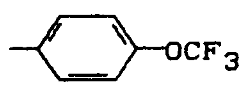 |
| 4-F | H | O | H | H | H | H | 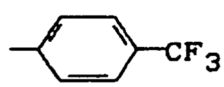 |
| 4-F | H | O | H | H | H | H | 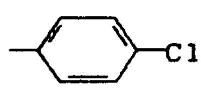 |

| Y | Z | X | $R^2$ | $R^3$ | $R^4$ | $R^5$ | $R^6$ |
|---|---|---|---|---|---|---|---|
| 4-F | H | O | $CH_3$ | H | H | H | 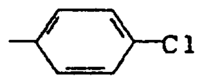 |
| 4-F | H | O | $-COOCH_3$ | | H | H | 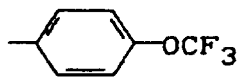 |
| 4-F | H | S | H | H | H | H | 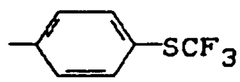 |
| 4-F | H | O | H | $CH_3$ | H | H | 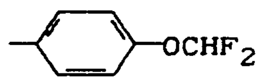 |
| 4-Cl | H | O | H | H | H | H | 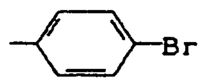 |
| 4-Cl | H | O | H | H | H | H | 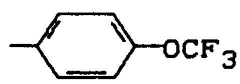 |
| 4-Cl | H | O | H | H | H | H | 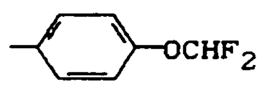 |
| 4-Cl | H | O | H | H | H | H | 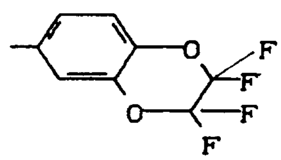 |
| $4-OCHF_2$ | H | O | H | H | H | H | 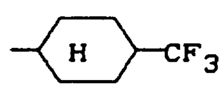 |
| $4-OCHF_2$ | H | O | H | H | H | H | 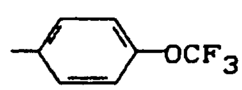 |

Tabelle 1D:

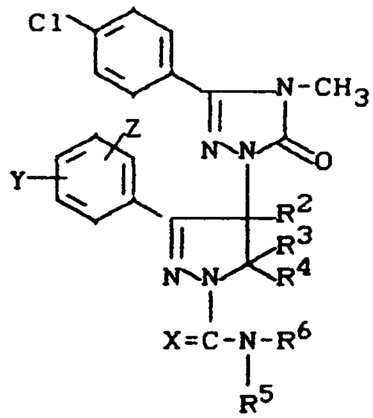

| Y | Z | X | $R^2$ | $R^3$ | $R^4$ | $R^5$ | $R^6$ |
|---|---|---|---|---|---|---|---|
| 4-$OCHF_2$ | H | O | H | H | H | H | 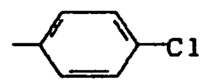 |
| 4-$OCHF_2$ | H | O | H | H | H | H | 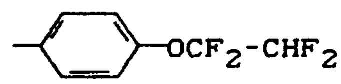 |
| 4-$OCH_2CF_3$ | H | O | H | H | H | H | 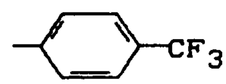 |
| 4-$OCH_2CF_3$ | H | O | H | H | H | H | 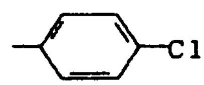 |
| 4-$CF_3$ | H | O | H | H | H | H | 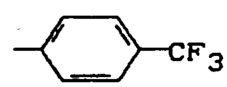 |
| 4-$CF_3$ | H | O | H | H | H | H | 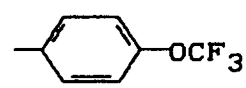 |

| Y | Z | X | $R^2$ | $R^3$ | $R^4$ | $R^5$ | $R^6$ |
|---|---|---|---|---|---|---|---|
| H | H | O | H | H | H | H | 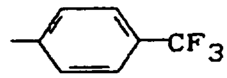 |
| 4-t.-Butyl | H | O | H | H | H | H | 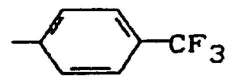 |
| 3-$CF_3$ | H | O | H | H | H | H | 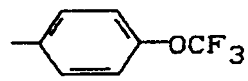 |
| 4-$CH_3$ | H | O | H | H | H | H | 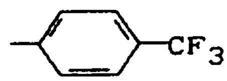 |
| 4-$CH_3$ | H | O | H | H | H | H | 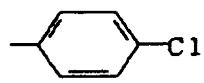 |
| 4-Cl | H | O | H | H | H | H | 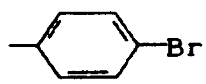 |
| 4-Cl | H | O | H | H | H | H | 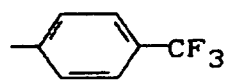 |
| 4-F | H | O | H | H | H | H | 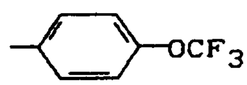 |
| 4-Cl | H | O | H | $CH_3$ | H | H | 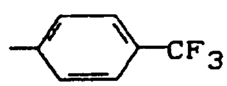 |
| 4-$OCHF_2$ | H | O | H | $CH_3$ | H | H | 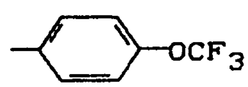 |
| 4-F | H | O | H | $CH_3$ | $CH_3$ | H | 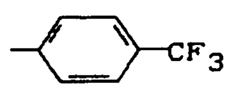 |

| Y | Z | X | $R^2$ | $R^3$ | $R^4$ | $R^5$ | $R^6$ |
|---|---|---|---|---|---|---|---|
| 4-$CF_3$ | H | O | H | H | H | H | 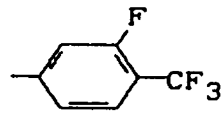 |
| 4-Cl | H | O | H | H | H | H | 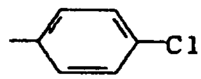 |
| 4-Br | H | O | H | H | H | H | 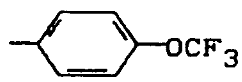 |
| 4-Cl | H | O | H | H | H | H | 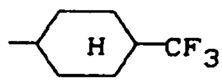 |

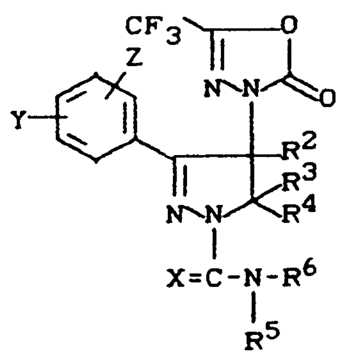

Tabelle 1E:

| Y | Z | X | $R^2$ | $R^3$ | $R^4$ | $R^5$ | $R^6$ |
|---|---|---|---|---|---|---|---|
| H | H | O | H | H | H | H | 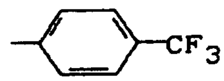 |
| H | H | O | H | H | H | H | 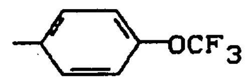 |
| 4-$CF_3$ | H | O | H | H | H | H | 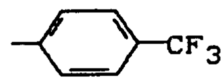 |
| 4-$CF_3$ | H | O | H | H | H | H | 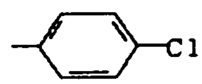 |
| 4-$CF_3$ | H | O | H | H | H | H | 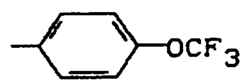 |
| 4-Br | H | O | H | H | H | H | 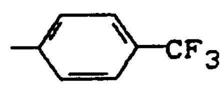 |
| 3-$CF_3$ | H | O | H | H | H | H | 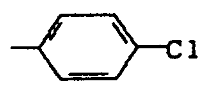 |
| 3-$CF_3$ | H | O | H | H | H | H | 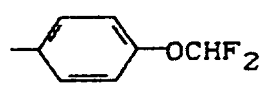 |

| Y | Z | X | $R^2$ | $R^3$ | $R^4$ | $R^5$ | $R^6$ |
|---|---|---|---|---|---|---|---|
| 4-F | H | O | H | H | H | H | 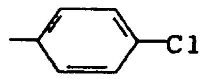 |
| 4-F | H | O | H | H | H | H | 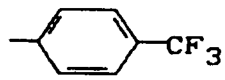 |
| 4-F | H | O | H | H | H | H | 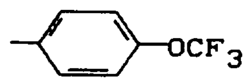 |
| 4-F | H | O | H | H | H | H | 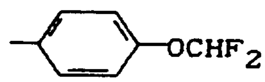 |
| 4-F | H | O | H | H | H | H | 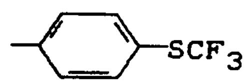 |
| 4-F | H | O | H | H | H | H | 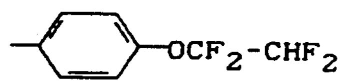 |
| 4-F | H | O | H | H | H | H | 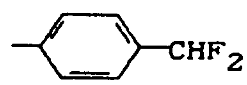 |
| 4-F | H | O | H | H | H | H | 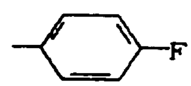 |
| 4-Cl | H | O | H | H | H | H | 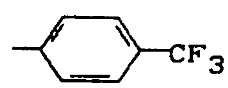 |
| 4-$OCHF_2$ | H | O | H | H | H | H | 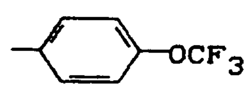 |
| 4-F | H | S | H | H | H | H | 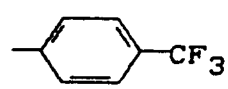 |

| Y | Z | X | $R^2$ | $R^3$ | $R^4$ | $R^5$ | $R^6$ |
|---|---|---|---|---|---|---|---|
| 4-F | H | O | $CH_3$ | H | H | H | 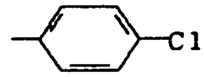 |
| 4-F | H | O | $COOCH_3$ | H | H | H | 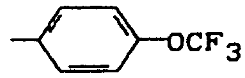 |
| 4-F | H | O | H | $CH_3$ | H | H | 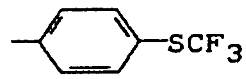 |
| 4-F | H | S | H | H | H | H | 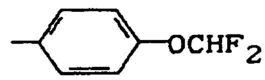 |
| 4-Cl | 3-Cl | O | H | H | H | H | 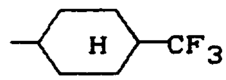 |
| 4-Cl | H | O | H | H | H | H | 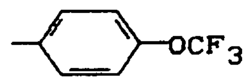 |
| 4-Cl | H | O | H | H | H | H | 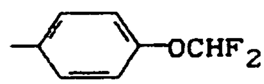 |
| 4-Cl | H | O | H | H | H | H | 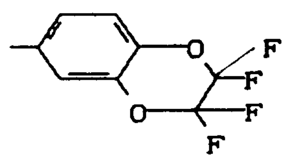 |
| 4-$OCHF_2$ | H | O | H | H | H | H | 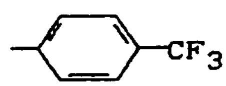 |
| 4-$OCHF_2$ | H | O | H | H | H | H | 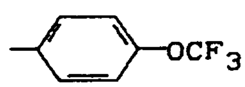 |

Tabelle 1F:

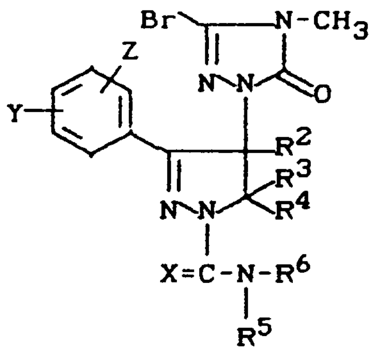

| Y | Z | X | $R^2$ | $R^3$ | $R^4$ | $R^5$ | $R^6$ |
|---|---|---|---|---|---|---|---|
| 4-$OCHF_2$ | H | O | H | H | H | H | 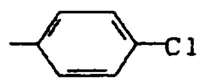 |
| 4-$OCHF_2$ | H | O | H | H | H | H | 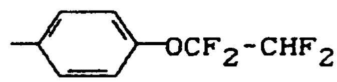 |
| 4-$OCH_2CF_3$ | H | O | H | H | H | H | 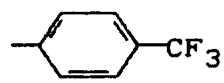 |
| 4-$OCH_2CF_3$ | H | O | H | H | H | H | 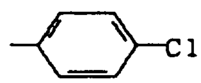 |
| 4-$CF_3$ | H | O | H | H | H | H | 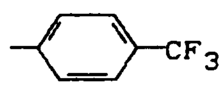 |
| 4-$CF_3$ | H | O | H | H | H | H | 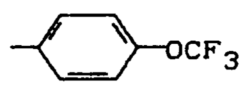 |
| H | H | O | H | H | H | H | 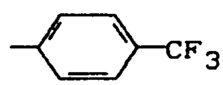 |
| 4-t.-Butyl | H | O | H | H | H | H | 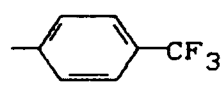 |

| Y | Z | X | $R^2$ | $R^3$ | $R^4$ | $R^5$ | $R^6$ |
|---|---|---|---|---|---|---|---|
| 3-$CF_3$ | H | O | H | H | H | H | 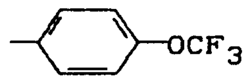 |
| 4-$CH_3$ | H | O | H | H | H | H | 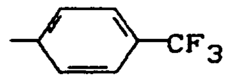 |
| 4-$CH_3$ | H | O | H | H | H | H | 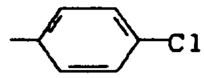 |
| 4-Cl | H | O | H | H | H | H | 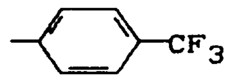 |
| 4-Cl | H | O | H | H | H | H | 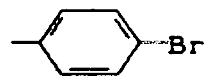 |
| 4-F | H | O | H | H | H | H | 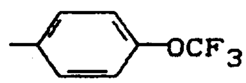 |
| 4-Cl | H | O | H | $CH_3$ | H | H | 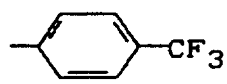 |
| 4-$OCHF_2$ | H | O | H | $CH_3$ | H | H | 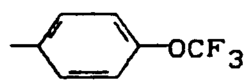 |
| 4-F | H | O | H | $CH_3$ | $CH_3$ | H | 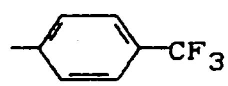 |
| 4-$CF_3$ | H | O | H | H | H | H | 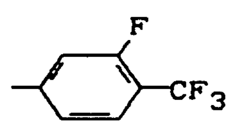 |
| 4-Cl | H | O | H | H | H | H | 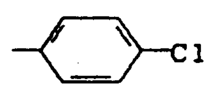 |

| Y | Z | X | $R^2$ | $R^3$ | $R^4$ | $R^5$ | $R^6$ |
|---|---|---|---|---|---|---|---|
| 4-Br | H | O | H | H | H | H | 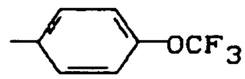 |
| 4-Cl | H | O | H | H | H | H | 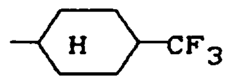 |

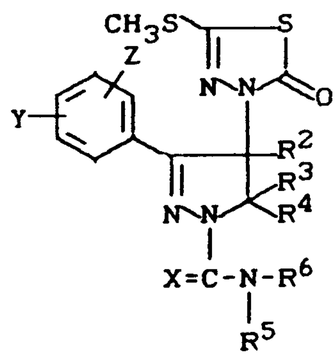

<u>Tabelle 1G</u>:

| Y | Z | X | $R^2$ | $R^3$ | $R^4$ | $R^5$ | $R^6$ |
|---|---|---|---|---|---|---|---|
| H | H | O | H | H | H | H | 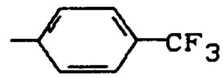 |
| H | H | O | H | H | H | H | 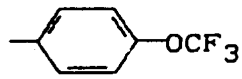 |
| $4-CF_3$ | H | O | H | H | H | H | 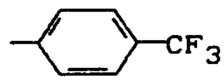 |
| $4-CF_3$ | H | O | H | H | H | H | 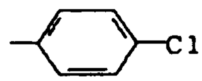 |
| $4-CF_3$ | H | O | H | H | H | H | 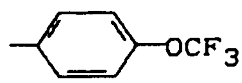 |
| 4-Br | H | O | H | H | H | H | 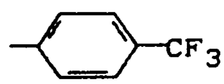 |
| $3-CF_3$ | H | O | H | H | H | H | 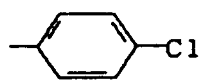 |
| $3-CF_3$ | H | O | H | H | H | H | 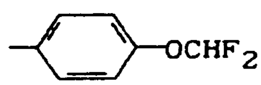 |

| Y | Z | X | $R^2$ | $R^3$ | $R^4$ | $R^5$ | $R^6$ |
|---|---|---|---|---|---|---|---|
| 4-F | H | O | H | H | H | H | 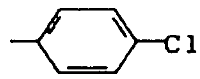 |
| 4-F | H | O | H | H | H | H | 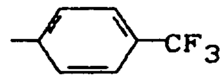 |
| 4-F | H | O | H | H | H | H | 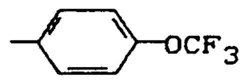 |
| 4-F | H | O | H | H | H | H | 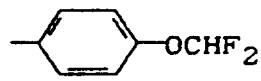 |
| 4-F | H | O | H | H | H | H | 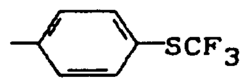 |
| 4-F | H | O | H | H | H | H | 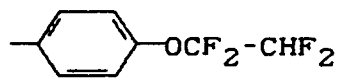 |
| 4-F | H | O | H | H | H | H | 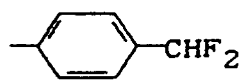 |
| 4-F | H | O | H | H | H | H | 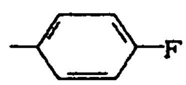 |
| 4-Cl | H | O | H | H | H | H | 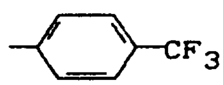 |
| 4-$OCHF_2$ | H | O | H | H | H | H | 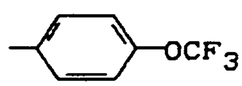 |
| 4-F | H | S | H | H | H | H | 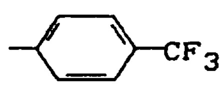 |

Tabelle 1H:

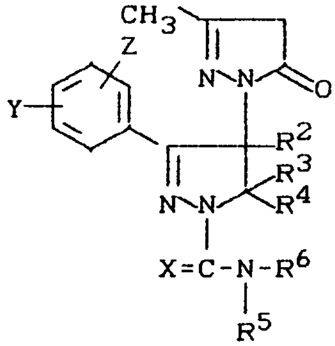

| Y | Z | X | $R^2$ | $R^3$ | $R^4$ | $R^5$ | $R^6$ |
|---|---|---|---|---|---|---|---|
| 4-F | H | O | $CH_3$ | H | H | H | 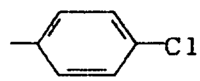 |
| 4-F | H | O | $COOCH_3$ | H | H | H | 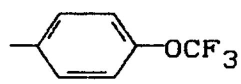 |
| 4-F | H | O | H | H | H | H | 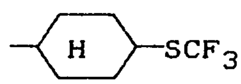 |
| 4-F | H | O | H | $CH_3$ | H | H | 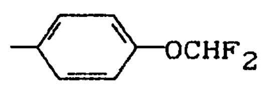 |
| 4-Cl | 3-Cl | O | H | H | H | H | 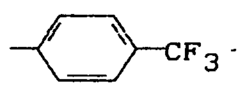 |
| 4-Cl | H | O | H | H | H | H | 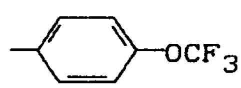 |
| 4-Cl | H | O | H | H | H | H | 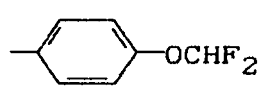 |
| 4-Cl | H | O | H | H | H | H | 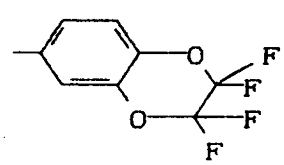 |

| Y | Z | X | $R^2$ | $R^3$ | $R^4$ | $R^5$ | $R^6$ |
|---|---|---|---|---|---|---|---|
| 4-$OCHF_2$ | H | O | H | H | H | H | 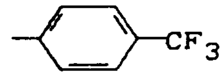 |
| 4-$OCHF_2$ | H | O | H | H | H | H | 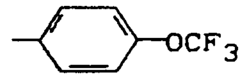 |
| 4-$OCHF_2$ | H | O | H | H | H | H | 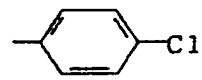 |
| 4-$OCHF_2$ | H | O | H | H | H | H | 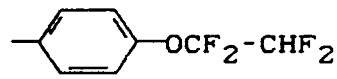 |
| 4-$OCH_2CF_3$ | H | O | H | H | H | H | 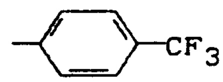 |
| 4-$OCH_2CF_3$ | H | O | H | H | H | H | 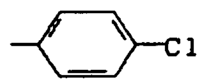 |
| 4-$CF_3$ | H | O | H | H | H | H | 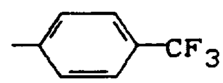 |
| 4-$CF_3$ | H | O | H | H | H | H | 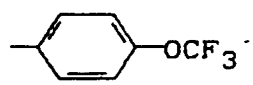 |
| H | H | O | H | H | H | H | 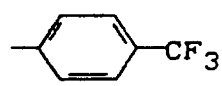 |
| 4-t.-Butyl | H | O | H | H | H | H | 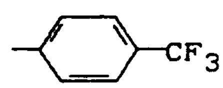 |
| 3-$CF_3$ | H | O | H | H | H | H | 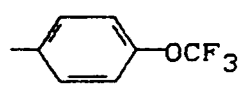 |

| Y | Z | X | $R^2$ | $R^3$ | $R^4$ | $R^5$ | $R^6$ |
|---|---|---|---|---|---|---|---|
| 4-$CH_3$ | H | O | H | H | H | H | 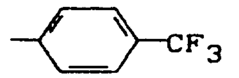 |
| 4-$SCH_3$ | H | O | H | H | H | H | 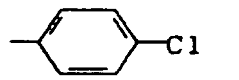 |
| 4-Br | H | O | H | H | H | H | 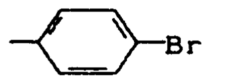 |
| 4-Cl | H | O | H | H | H | H | 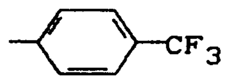 |
| 4-F | H | O | H | H | H | H | 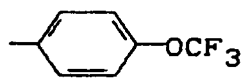 |
| 4-Cl | H | O | H | $CH_3$ | H | H | 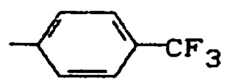 |
| 4-$OCHF_2$ | H | O | H | $CH_3$ | H | H | 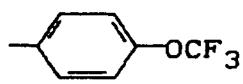 |
| 4-F | H | O | H | $CH_3$ | $CH_3$ | H | 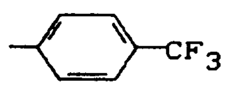 |
| 4-$CF_3$ | H | O | H | H | H | H | 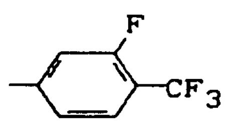 |
| 4-Cl | H | O | H | H | H | H | 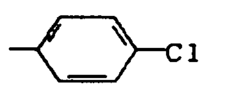 |
| 4-Br | H | O | H | H | H | H | 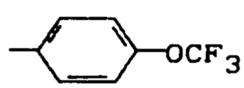 |
| 4-F | H | O | H | H | H | H | 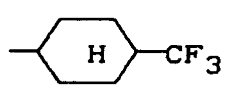 |

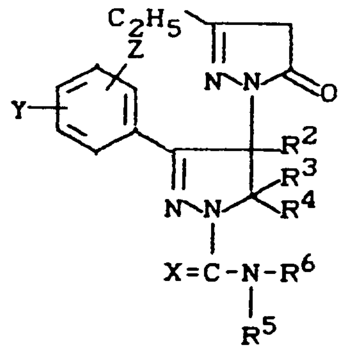

Tabelle 1I:

| Y | Z | X | $R^2$ | $R^3$ | $R^4$ | $R^5$ | $R^6$ |
|---|---|---|---|---|---|---|---|
| H | H | O | H | H | H | H | 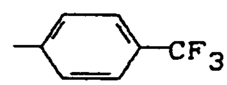 |
| H | H | O | H | H | H | H | 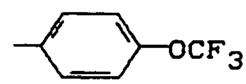 |
| 4-$CF_3$ | H | O | H | H | H | H | 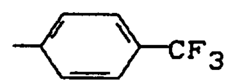 |
| 4-$CF_3$ | H | O | H | H | H | H | 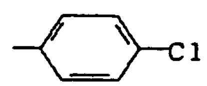 |
| 4-$CF_3$ | H | O | H | H | H | H | 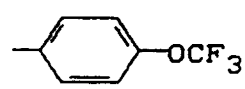 |
| 4-Br | H | O | H | H | H | H | 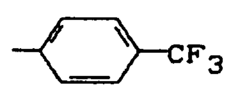 |
| 3-$CF_3$ | H | O | H | H | H | H | 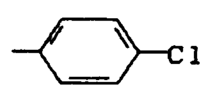 |
| 3-$CF_3$ | H | O | H | H | H | H | 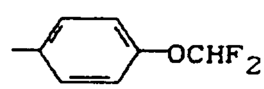 |

| Y | Z | X | $R^2$ | $R^3$ | $R^4$ | $R^5$ | $R^6$ |
|---|---|---|---|---|---|---|---|
| 4-F | H | O | H | H | H | H | 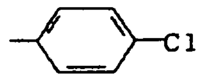 |
| 4-F | H | O | H | H | H | H | 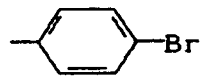 |
| 4-F | H | O | H | H | H | H | 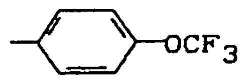 |
| 4-F | H | O | H | H | H | H | 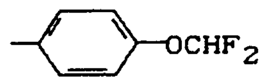 |
| 4-F | H | O | H | H | H | H | 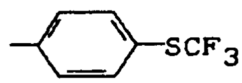 |
| 4-F | H | O | H | H | H | H | 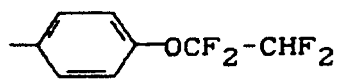 |
| 4-F | H | O | H | H | H | H | 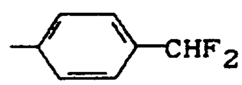 |
| 4-F | H | O | H | H | H | H | 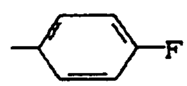 |
| 4-Cl | H | O | H | H | H | H | 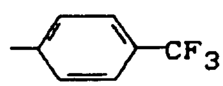 |
| 4-$OCHF_2$ | H | O | H | H | H | H | 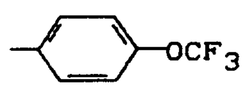 |
| 4-F | H | O | H | H | H | H | 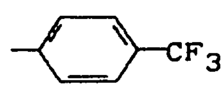 |

| Y | Z | X | $R^2$ | $R^3$ | $R^4$ | $R^5$ | $R^6$ |
|---|---|---|---|---|---|---|---|
| 3,4-O-$CF_2$-O- | | O | H | H | H | H | 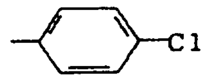 |
| 4-Cl | H | O | H | H | H | H | 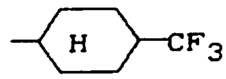 |
| 4-F | H | O | H | H | H | H | 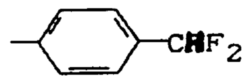 |
| 4-F | H | O | H | H | H | H | 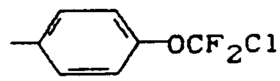 |
| 4-Cl | H | O | $-COOCH_3$ | H | H | H | 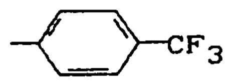 |
| 4-Cl | H | O | H | H | H | H | 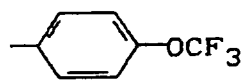 |
| 4-Cl | H | O | H | H | H | H | 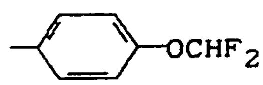 |
| 4-Cl | H | O | H | H | H | H | 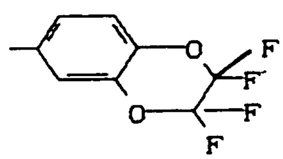 |
| 4-$OCHF_2$ | H | O | H | H | H | H | 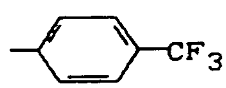 |
| 4-$OCHF_2$ | H | O | H | H | H | H | 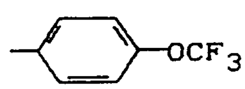 |

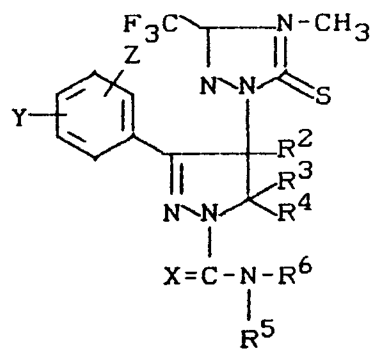

Tabelle 1K:

| Y | Z | X | $R^2$ | $R^3$ | $R^4$ | $R^5$ | $R^6$ |
|---|---|---|---|---|---|---|---|
| 4-$OCHF_2$ | H | O | H | H | H | H | 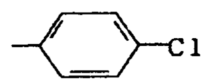 |
| 4-$OCHF_2$ | H | O | H | H | H | H | 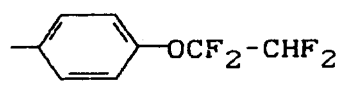 |
| 4-$OCH_2CF_3$ | H | O | H | H | H | H | 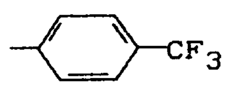 |
| 4-$OCH_2CF_3$ | H | O | H | H | H | H | 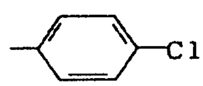 |
| 4-$CF_3$ | H | O | H | H | H | H | 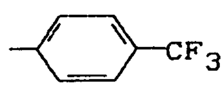 |
| 4-$CF_3$ | H | O | H | H | H | H | 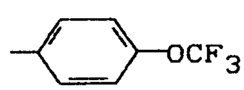 |
| H | H | O | H | H | H | H | 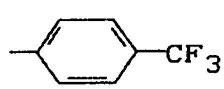 |
| 4-t.-Butyl | H | O | H | H | H | H | 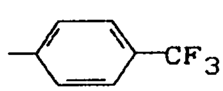 |

| Y | Z | X | $R^2$ | $R^3$ | $R^4$ | $R^5$ | $R^6$ |
|---|---|---|---|---|---|---|---|
| $3-CF_3$ | H | O | H | H | H | H | 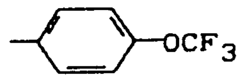 |
| $4-CH_3$ | H | O | H | H | H | H | 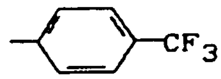 |
| $4-CH_3$ | H | O | H | H | H | H | 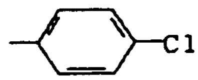 |
| 4-Cl | H | O | H | H | H | H | 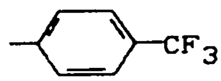 |
| 4-Cl | H | O | H | H | H | H | 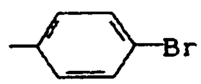 |
| 4-F | H | O | H | H | H | H | 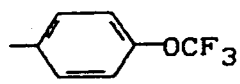 |
| 4-Cl | H | O | H | $CH_3$ | H | H | 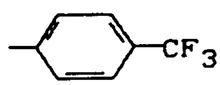 |
| 4-OCHF 2 | H | O | H | $CH_3$ | H | H | 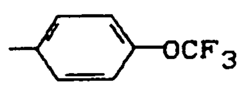 |
| 4-F | H | O | H | $CH_3$ | $CH_3$ | H | 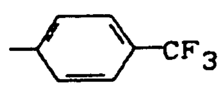 |
| $4-CF_3$ | H | O | H | H | H | H | 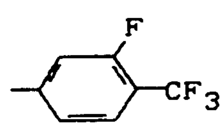 |

| Y | Z | X | $R^2$ | $R^3$ | $R^4$ | $R^5$ | $R^6$ |
|---|---|---|---|---|---|---|---|
| 4-Cl | H | O | H | H | H | H | 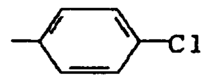 |
| 4-Br | H | O | H | H | H | H | 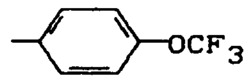 |
| 4-Cl | H | O | H | H | H | H | 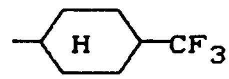 |

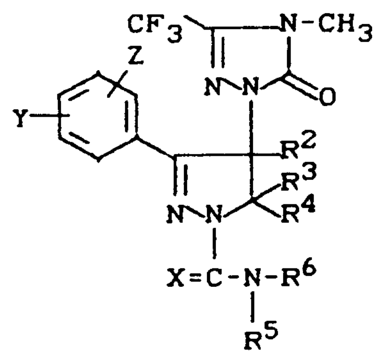

<u>Tabelle 1L</u>:

| Y | Z | X | $R^2$ | $R^3$ | $R^4$ | $R^5$ | $R^6$ |
|---|---|---|---|---|---|---|---|
| H | H | O | H | H | H | H | 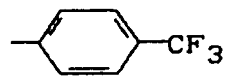 |
| H | H | O | H | H | H | H | 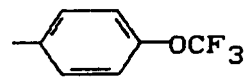 |
| 4-$CF_3$ | H | O | H | H | H | H | 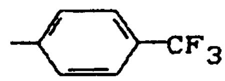 |
| 4-$CF_3$ | H | O | H | H | H | H | 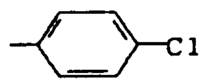 |
| 4-$CF_3$ | H | O | H | H | H | H | 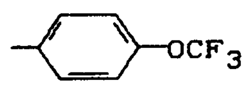 |
| 4-Br | H | O | H | H | H | H | 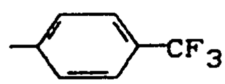 |
| 3-$CF_3$ | H | O | H | H | H | H | 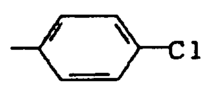 |
| 3-$CF_3$ | H | O | H | H | H | H | 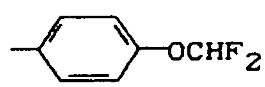 |

| Y | Z | X | $R^2$ | $R^3$ | $R^4$ | $R^5$ | $R^6$ |
|---|---|---|---|---|---|---|---|
| 4-F | H | O | H | H | H | H | 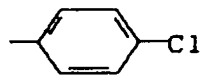 |
| 4-F | H | O | H | H | H | H | 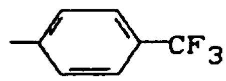 |
| 4-F | H | O | H | H | H | H | 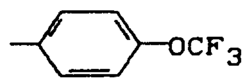 |
| 4-F | H | O | H | H | H | H | 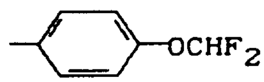 |
| 4-F | H | O | H | H | H | H | 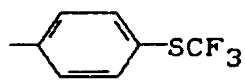 |
| 4-F | H | O | H | H | H | H | 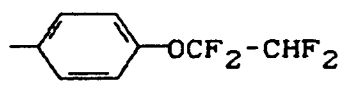 |
| 4-F | H | O | H | H | H | H | 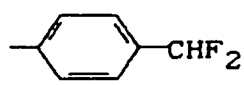 |
| 4-F | H | O | H | H | H | H | 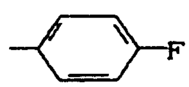 |
| 4-Cl | H | O | H | H | H | H | 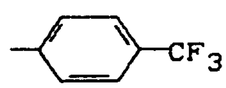 |
| 4-$OCHF_2$ | H | O | H | H | H | H | 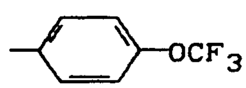 |
| 4-F | H | O | $-CH_3$ | H | H | H | 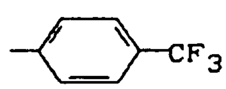 |

| Y | Z | X | $R^2$ | $R^3$ | $R^4$ | $R^5$ | $R^6$ |
|---|---|---|---|---|---|---|---|
| 4-O-$(CH_2)_2$-$CH_3$ | H | O | H | H | H | H | 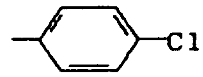 |
| 4-F | H | O | $CH_3$ | H | H | H | 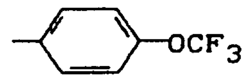 |
| 4-F | H | O | $COOCH_3$ | H | H | H | 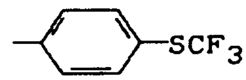 |
| 4-F | H | O | H | H | H | H | 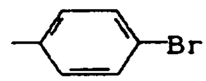 |
| 4-Cl | H | O | H | H | H | H | 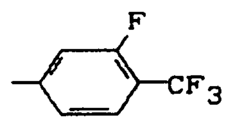 |
| 4-Cl | H | O | H | H | H | H | 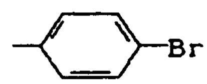 |
| 4-Cl | H | O | H | H | H | H | 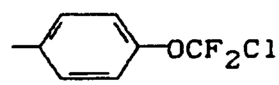 |
| 4-Cl | H | O | H | H | H | H | 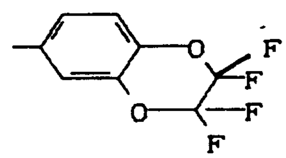 |
| 4-$OCHF_2$ | H | S | H | H | H | H | 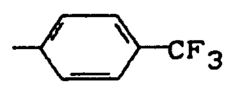 |
| 4-$OCHF_2$ | H | O | H | H | H | H | 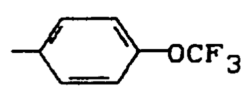 |

| Y | Z | X | $R^2$ | $R^3$ | $R^4$ | $R^5$ | $R^6$ |
|---|---|---|---|---|---|---|---|
| 4-$OCHF_2$ | H | O | H | H | H | H | 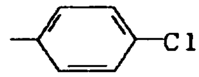 |
| 4-$OCHF_2$ | H | O | H | H | H | H | 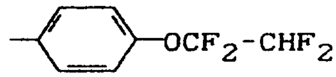 |
| 4-$OCH_2CF_3$ | H | O | H | H | H | H | 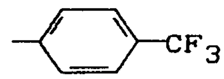 |
| 4-$OCH_2CF_3$ | H | O | H | H | H | H | 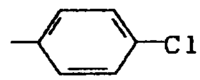 |
| 4-Br | H | O | H | H | H | H | 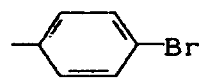 |
| 4-Cl | H | O | H | H | H | H | 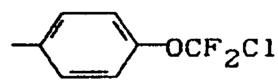 |
| H | H | O | H | H | H | H | 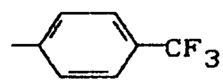 |
| 4-t.-Butyl | H | O | H | H | H | H | 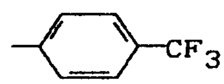 |
| 3-$CF_3$ | H | O | H | H | H | H | 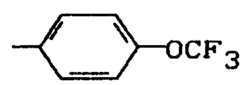 |
| 4-$CH_3$ | H | O | H | H | H | H | 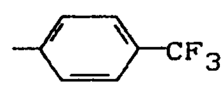 |
| 4-$CH_3$ | H | O | H | H | H | H | 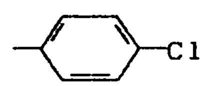 |

| Y | Z | X | $R^2$ | $R^3$ | $R^4$ | $R^5$ | $R^6$ |
|---|---|---|---|---|---|---|---|
| 4-Cl | H | O | H | $CH_3$ | H | H | 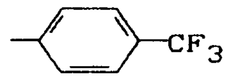 |
| 4-Cl | H | O | H | H | H | H | 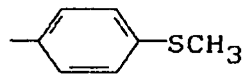 |
| 4-Br | H | O | H | H | H | H | 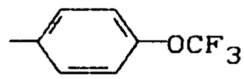 |
| 4-O-$(CH_2)_2$-$CH_3$ | H | O | H | $CH_3$ | H | H | 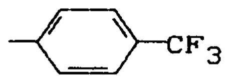 |
| 4-OCHF | 2 H | O | H | $CH_3$ | H | H | 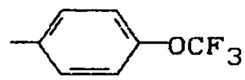 |
| 4-F | H | O | H | $CH_3$ | $CH_3$ | H | 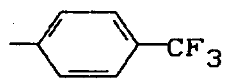 |
| 4-$CF_3$ | H | O | H | H | H | H | 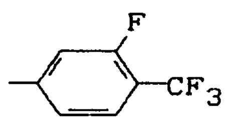 |
| 4-Cl | H | O | H | H | H | H | 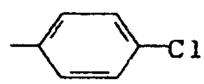 |
| 4-Cl | H | O | H | H | H | H | 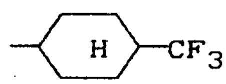 |

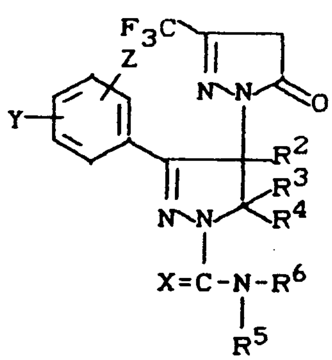

Tabelle M:

| Y | Z | X | $R^2$ | $R^3$ | $R^4$ | $R^5$ | $R^6$ |
|---|---|---|---|---|---|---|---|
| H | H | O | H | H | H | H | 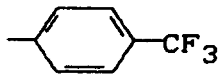 |
| H | H | O | H | H | H | H | 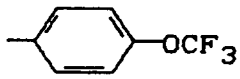 |
| 4-$CF_3$ | H | O | H | H | H | H | 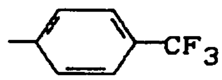 |
| H | H | O | H | H | H | H | 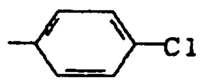 |
| 4-$CF_3$ | H | O | H | H | H | H | 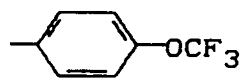 |
| 4-Br | H | O | H | H | H | H | 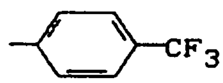 |
| 3-$CF_3$ | H | O | H | H | H | H | 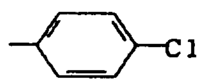 |
| 3-$CF_3$ | H | O | H | H | H | H | 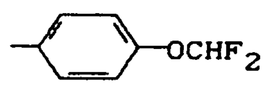 |

| Y | Z | X | $R^2$ | $R^3$ | $R^4$ | $R^5$ | $R^6$ |
|---|---|---|---|---|---|---|---|
| 4-F | H | O | H | H | H | H | 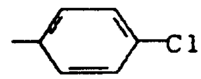 |
| 4-F | H | O | H | H | H | H | 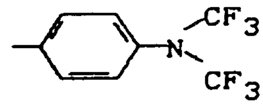 |
| 4-F | H | O | H | H | H | H | 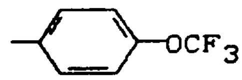 |
| 4-F | H | O | H | H | H | H | 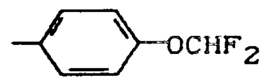 |
| 4-F | H | O | H | H | H | H | 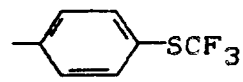 |
| 4-F | H | O | H | H | H | H | 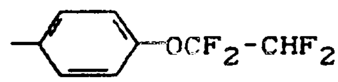 |
| 4-F | H | O | H | H | H | H | 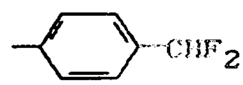 |
| 4-F | H | O | H | H | H | H | 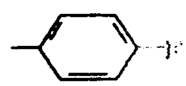 |
| 4-Cl | H | O | H | H | H | H | 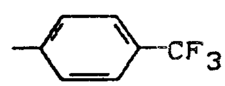 |
| 4-$OCHF_2$ | H | O | H | H | H | H | 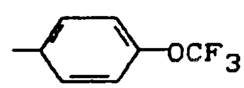 |
| 4-F | H | O | H | H | H | H | 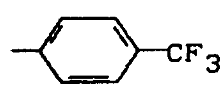 |

| Y | Z | X | $R^2$ | $R^3$ | $R^4$ | $R^5$ | $R^6$ |
|---|---|---|---|---|---|---|---|
| 4-Br | H | O | H | H | H | H | 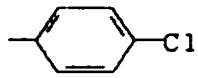 |
| 4-F | H | O | H | H | H | $-CH_3$ | 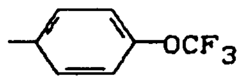 |
| 4-Br | H | O | H | H | H | H | 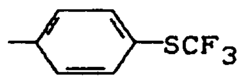 |
| 4-F | H | O | H | H | H | H | 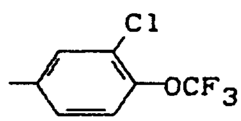 |
| 4-Cl | H | O | H | H | H | H | 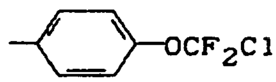 |
| 4-Cl | H | O | H | H | H | H | 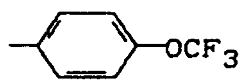 |
| 4-Cl | H | O | H | H | H | H | 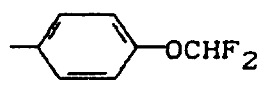 |
| 4-Cl | H | O | H | H | H | H | 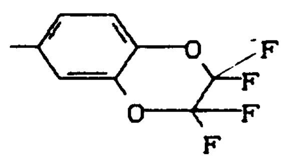 |
| 4-OCHF 2 | H | O | H | H | H | H | 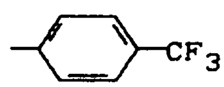 |
| $4-OCHF_2$ | H | O | H | H | H | H | 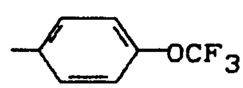 |

| Y | Z | X | $R^2$ | $R^3$ | $R^4$ | $R^5$ | $R^6$ |
|---|---|---|---|---|---|---|---|
| 4-$OCHF_2$ | H | O | H | H | H | H | 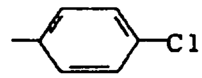 |
| 4-$OCHF_2$ | H | O | H | H | H | H | 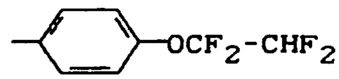 |
| 4-$OCH_2CF_3$ | H | O | H | H | H | H | 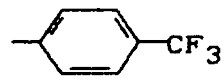 |
| 4-$OCH_2CF_3$ | H | O | H | H | H | H | 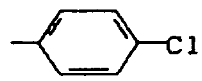 |
| 4-$CF_3$ | H | O | H | H | H | H | 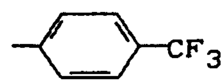 |
| 4-$CF_3$ | H | O | H | H | H | H | 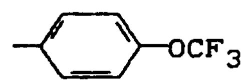 |
| 4-O-$CH_2$-$CH_3$ | H | O | H | H | H | H | 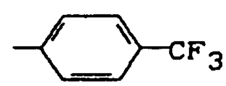 |
| 4-t.-Butyl | H | O | H | H | H | H | 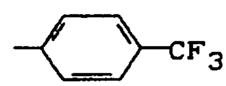 |
| 3-$CF_3$ | H | O | H | H | H | H | 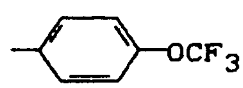 |
| 4-$CH_3$ | H | O | H | H | H | H | 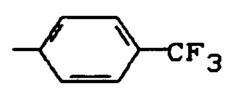 |
| 4-$CH_3$ | H | O | H | H | H | H | 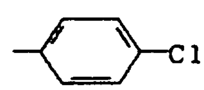 |

| Y | Z | X | $R^2$ | $R^3$ | $R^4$ | $R^5$ | $R^6$ |
|---|---|---|---|---|---|---|---|
| $-O-(CH_2)_2-CH_3$ | H | O | H | H | H | H | 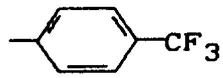 |
| 4-Cl | H | O | H | H | H | H | 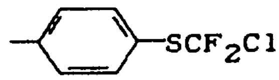 |
| 4-F | H | O | H | H | H | H | 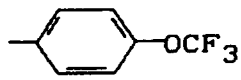 |
| 4-Cl | H | O | H | $CH_3$ | H | H | 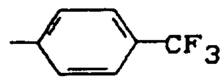 |
| 4-OCHF 2 | H | O | H | $CH_3$ | H | H | 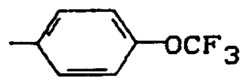 |
| 4-F | H | O | H | $CH_3$ | $CH_3$ | H | 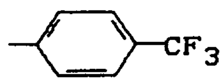 |
| $4-CF_3$ | H | O | H | H | H | H | 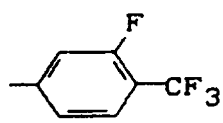 |
| 4-Cl | H | O | H | H | H | H | 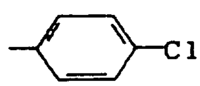 |
| 4-Br | H | O | H | H | H | H | 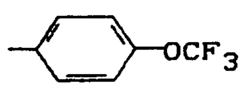 |
| 4-Cl | H | O | H | H | H | H | 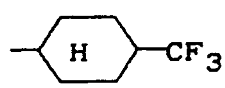 |

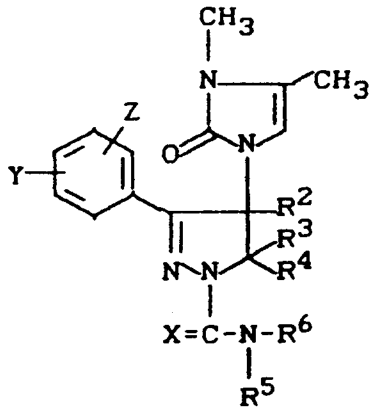

| Y | Z | X | $R^2$ | $R^3$ | $R^4$ | $R^5$ | $R^6$ |
|---|---|---|---|---|---|---|---|
| 4-$CF_3$ | H | O | H | H | H | H | 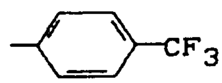 |
| 4-$CF_3$ | H | O | H | H | H | H | 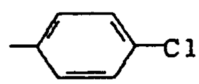 |
| 4-$CF_3$ | H | O | H | H | H | H | 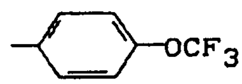 |
| 4-Br | H | O | H | H | H | H | 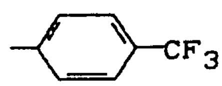 |
| 3-$CF_3$ | H | O | H | H | H | H | 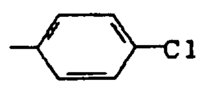 |
| 3-$CF_3$ | H | O | H | H | H | H | 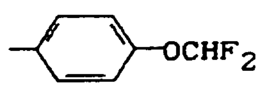 |
| 4-F | H | O | H | H | H | H | 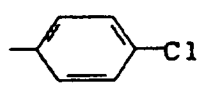 |

| Y | Z | X | $R^2$ | $R^3$ | $R^4$ | $R^5$ | $R^6$ |
|---|---|---|---|---|---|---|---|
| 4-F | H | O | H | H | H | H | 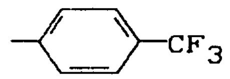 |
| 4-F | H | O | H | H | H | H | 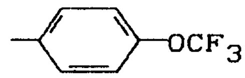 |
| 4-F | H | O | H | H | H | H | 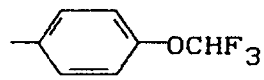 |
| 4-F | H | O | H | H | H | H | 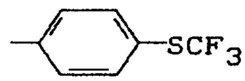 |
| H | H | O | H | H | H | H | 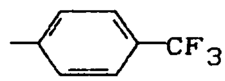 |
| H | H | O | H | H | H | H | 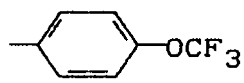 |
| 4-F | H | O | H | H | H | H | 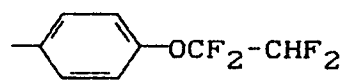 |
| 4-F | H | O | H | H | H | H | 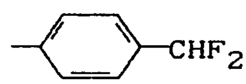 |
| 4-F | H | O | H | H | H | H | 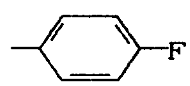 |
| 4-Cl | H | O | H | H | H | H | 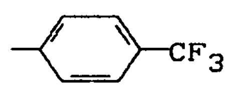 |
| 4-$OCHF_2$ | H | O | H | H | H | H | 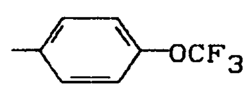 |
| 4-F | H | O | H | H | H | H | 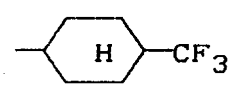 |

| Y | Z | X | $R^2$ | $R^3$ | $R^4$ | $R^5$ | $R^6$ |
|---|---|---|---|---|---|---|---|
| 4-F | H | O | $CH_3$ | H | H | H | 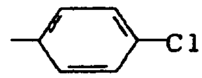 |
| 4-F | H | O | $COOCH_3$ | H | H | H | 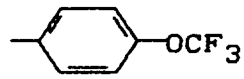 |
| 4-F | H | O | H | $CH_3$ | H | H | 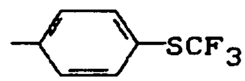 |
| 4-F | H | S | H | H | H | H | 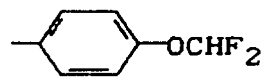 |
| 4-Cl | 3-Cl | O | H | H | H | H | 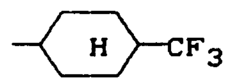 |
| 4-Cl | H | O | H | H | H | H | 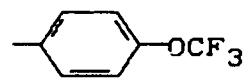 |
| 4-Cl | H | O | H | H | H | H | 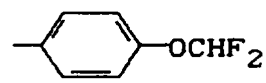 |
| 4-Cl | H | O | H | H | H | H | 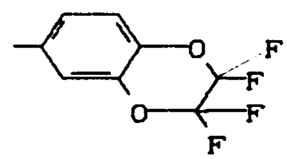 |
| 4-$OCHF_2$ | H | O | H | H | H | H | 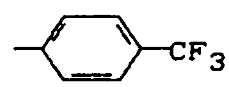 |
| 4-$OCHF_2$ | H | O | H | H | H | H | 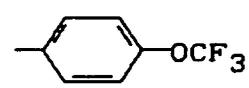 |

| Y | Z | X | $R^2$ | $R^3$ | $R^4$ | $R^5$ | $R^6$ |
|---|---|---|---|---|---|---|---|
| 4-$OCHF_2$ | H | O | H | H | H | H | 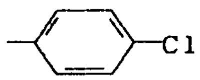 |
| 4-$OCHF_2$ | H | O | H | H | H | H | 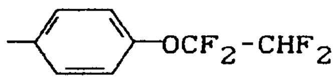 |
| 4-$OCH_2CF_3$ | H | O | H | H | H | H | 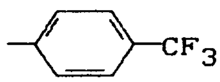 |
| 4-$OCH_2CF_3$ | H | O | H | H | H | H | 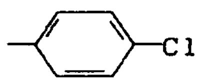 |
| 4-$CF_3$ | H | O | H | H | H | H | 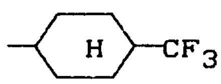 |
| 4-F | H | O | H | H | H | H | 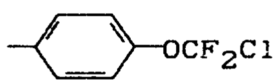 |
| 4-F | H | O | $CH_3$ | H | H | H | 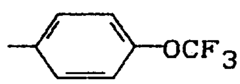 |
| 4-F | H | O | $COOCH_3$ | H | H | H | 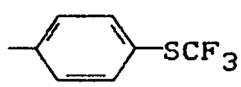 |
| 4-Cl | H | O | H | H | H | H | 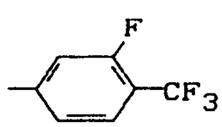 |

Verwendet man beispielsweise 3-(4'-Chlorphenyl)-4-(4''-methyl-3''-trifluormethyl-$\Delta^{2''}$-1'',2'',''4-triazolin-5''-on-1''-yl)-4,5-dihydropyrazol und 4-Trifluor-methoxy-phenyl-isocyanat als Ausgangsstoffe, so läßt sich der Reaktionsablauf des erfindungsgemäßen Verfahrens (A) durch das folgende Formelschema darstellen:

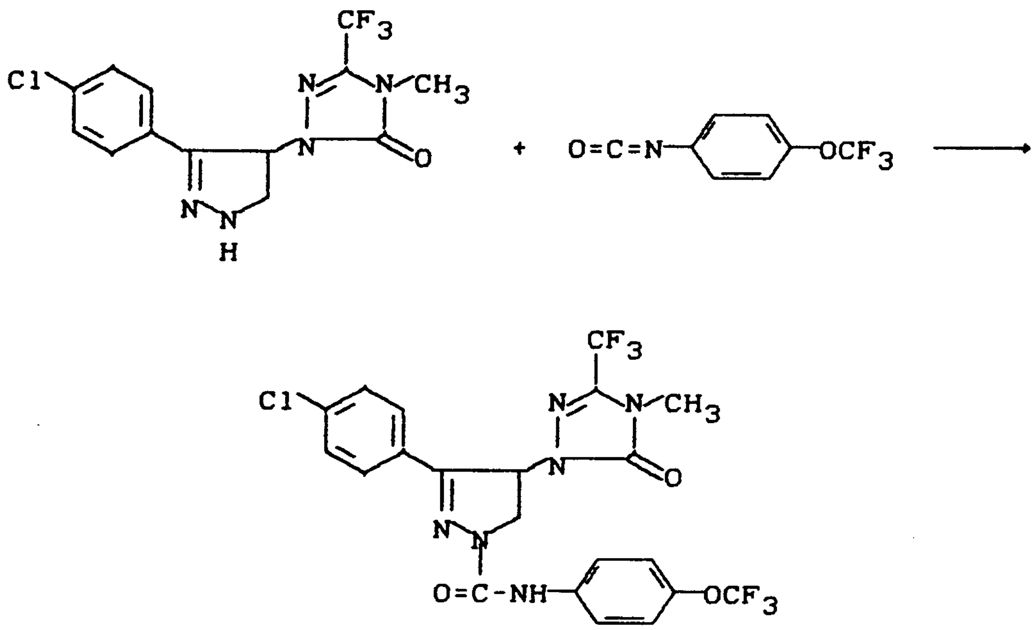

Verwendet man beispielsweise 3-(4'-Chlorphenyl)-4-(4''-methyl-3''-trifluormethyl-$\Delta^{2''}$-1'',2'',4''-triazolin-5''-on-1''-yl)-4,5-dihydro-1-pyrazol-carbonsäure-(4-trifluormethoxy)-anilid und 2-Jodpropan als Ausgangsstoffe, so kann der Reaktionsablauf des erfindungsgemäßen Verfahrens (B) wie folgt dargestellt werden:

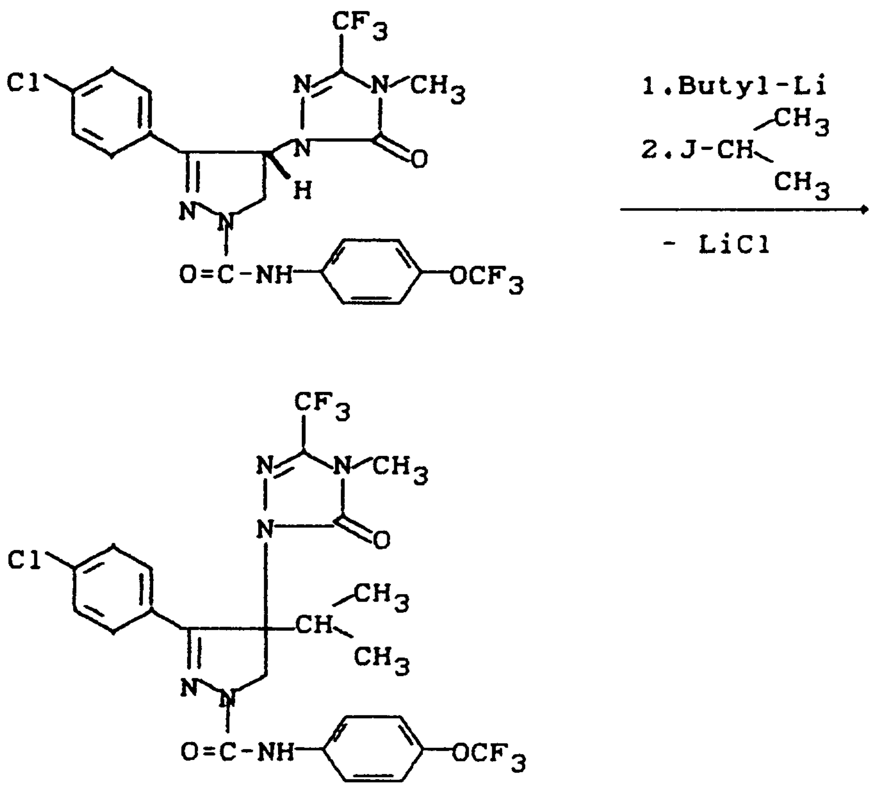


Die zur Durchführung des erfindungsgemäßen Verfahrens (A) als Ausgangsstoffe benötigten Pyrazolinderivate sind durch die Formel (II) allgemein definiert. In dieser Formel (II) stehen $R^1$, $R^2$, $R^3$, $R^4$, Y und Z vorzugsweise für diejenigen Reste, die bereits im Zusammenhang mit der Beschreibung der erfindungsgemäßen Stoffe der Formel (I) als bevorzugt für diese Substituenten genannt wurden. Die Pyrazolinderivate der Formel (II) sind neu und können nach einem der folgenden Verfahren hergestellt werden.

Sie werden durch Umsetzung von Verbindungen der Formel (VI)

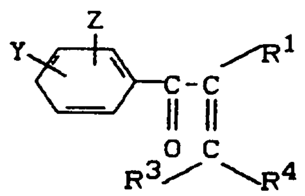

(VI)

in einem polaren organischen Lösungsmittel, vorzugsweise einem Alkanol bei Temperaturen von 20 bis 80 ° C insbesondere bei 30 bis 60 ° C mit Hydrazin-Hydrat hergestellt:

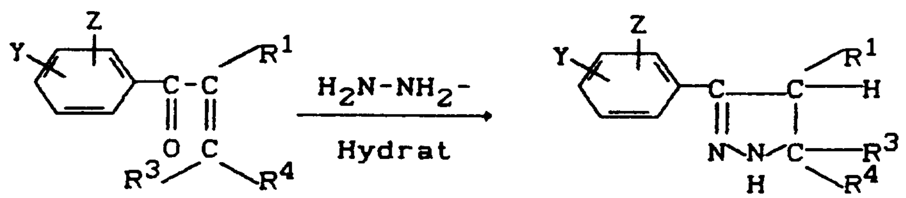


(VI) (II)

In Abhängigkit von der Bedeutung der Substituenten $R^3$ und $R^4$ ergeben sich dabei folgende Herstellungsvarianten der Ausgangsverbindungen der Formel (VI)

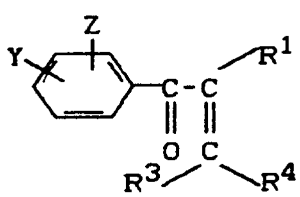

(VI)

a) $R^3$ und $R^4$ in der Formel (VI) stehen für Wasserstoff

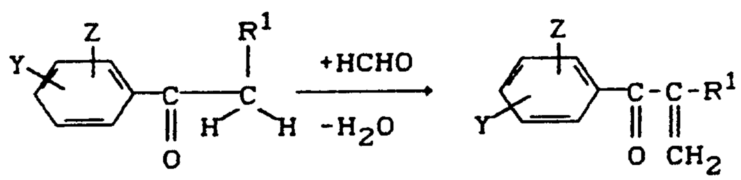


(VIa) (VIb)

Hierbei wird in einem polaren organischen Lösungsmittel, vorzugsweise einem Alkanol und insbesondere in Ethanol oder Methanol mit einer Formalinlösung unter Zusatz geringer Mengen einer organischen Base, insbesondere von Piperidin sowie Zusatz von Eisessig umgesetzt.

b) In der Formel (VI) steht $R^3$ für Alkyl oder Aryl und $R^4$ steht für Wasserstoff:

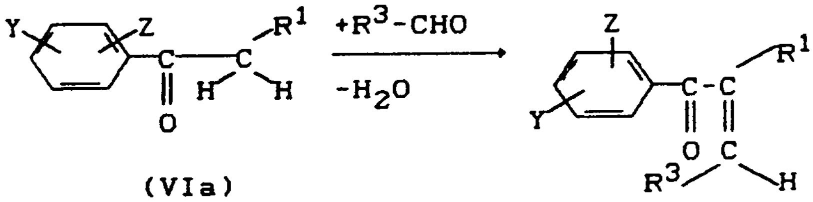


(VIa) (VIc)

Die Verfahrensbedingungen entsprechen denjenigen der Umsetzung mit Formaldehyd.

c) In der Formel (VI) stehen $R^3$ und $R^4$ für Alkyl:

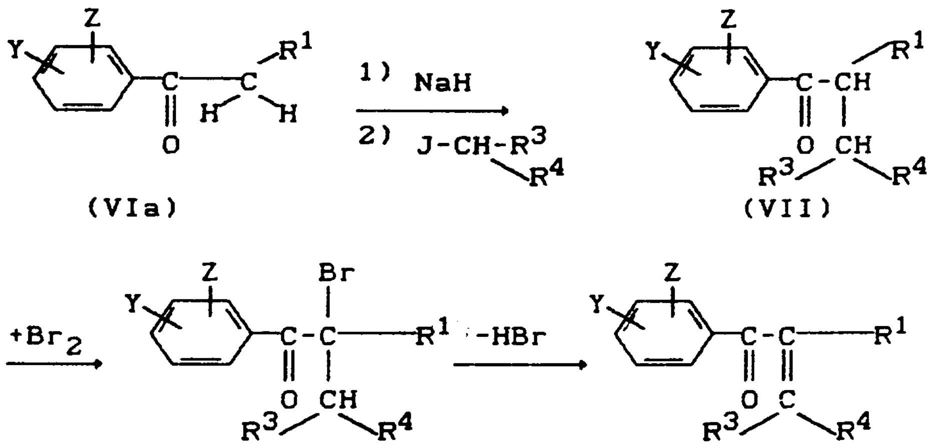


(VIa) (VII)

(VIII) (IX)

Hierbei wird die Verbindung (VI) zunächst mit einer starken Base in das Salz übergeführt und anschließend mit einem Halogenid, insbesondere einem Jodid der Formel

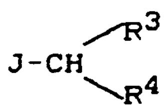

umgesetzt.

Die dabei entstehenden Verbindungen der Formel (VII) wird bromiert und anschließend wird durch Zusatz einer Base unter HBr-Eliminierung das Zwischenprodukt der Formel (IX) hergestellt.

Verbindungen der Formel (II), in welcher $R^3$ und $R^4$ für Wasserstoff stehen, werden außedem durch Umsetzung von Verbindungen der Formel (VId)

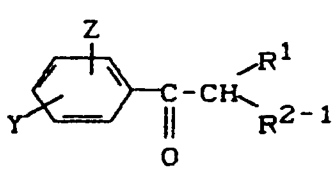

(VId)

in welcher

Y und Z die oben angegebene Bedeutung haben und

$R^{2-1}$ für Wasserstoff, Alkyl oder Alkoxycarbonyl steht,

erhalten, indem man diese zunächst in einer 1. Stufe in einem polaren organischen Lösungsmittel, vorzugsweise Acetonitril, bei Temperaturen von 10 bis 100 °C, insbesondere bei 20 bis 80 °C mit einem Mol N,N-Dimethylmethylenimmoniumchlorid der Formel (XI)

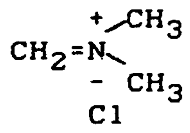

(XI)

gegebenenfalls am Rückfluß erhitzt, wobei das intermediär auftretende Zwischenprodukt der Formel (XII)

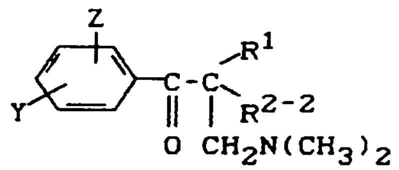

(XII)

gegebenenfalls isoliert und anschließend in einer zweiten Stufe in einem polaren organischen Lösungsmittel, vorzugsweise einem Alkohol bei Temperaturen von 20 bis 80 °C insbesondere bei 30 bis 60 °C mit Hydrazin-Hydrat zu Verbindungen der Formel (II) cyclisiert.

Verbindungen der Formel (II), in welcher $R^3$ für den Rest $-CH_2-N(CH_3)_2$ und $R^4$ für Wasserstoff steht, werden erhalten, indem man zunächst in einer 1. Stufe Verbindungen der Formel (VId)

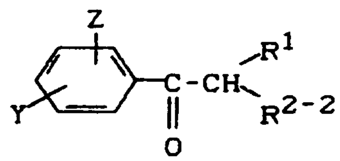

(VId)

in welcher

Y und Z die oben angegebene Bedeutung haben und

$R^{2-1}$ für Wasserstoff, Alkyl oder Alkoxycarbonyl steht,

in einem polaren organischen Lösungsmittel, vorzugsweise Acetonitril, bei Temperaturen von 10 bis 100 °C, insbesondere bei 20 bis 80 °C mit 2 Mol N,N-Dimethylmethylenimmoniumchlorid der Formel (XI)

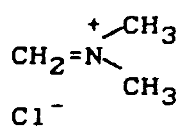

(XI)

gegebenenfalls am Rückfluß erhitzt, wobei das intermediär auftretende Zwischenprodukt der Formel (XIIa)

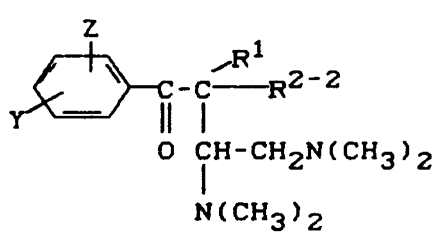

(XIIa)

gegebenenfalls isoliert und anschließend in einer zweiten Stufe in einem polaren organischen Lösungsmittel, vorzugsweise einem Alkohol bei Temperaturen von 20 bis 80 °C insbesondere bei 30 bis 60 °C mit Hydrazin-Hydrat zu Verbindungen der Formel (II) cyclisiert (vgl. auch das Herstellungsbeispiel).

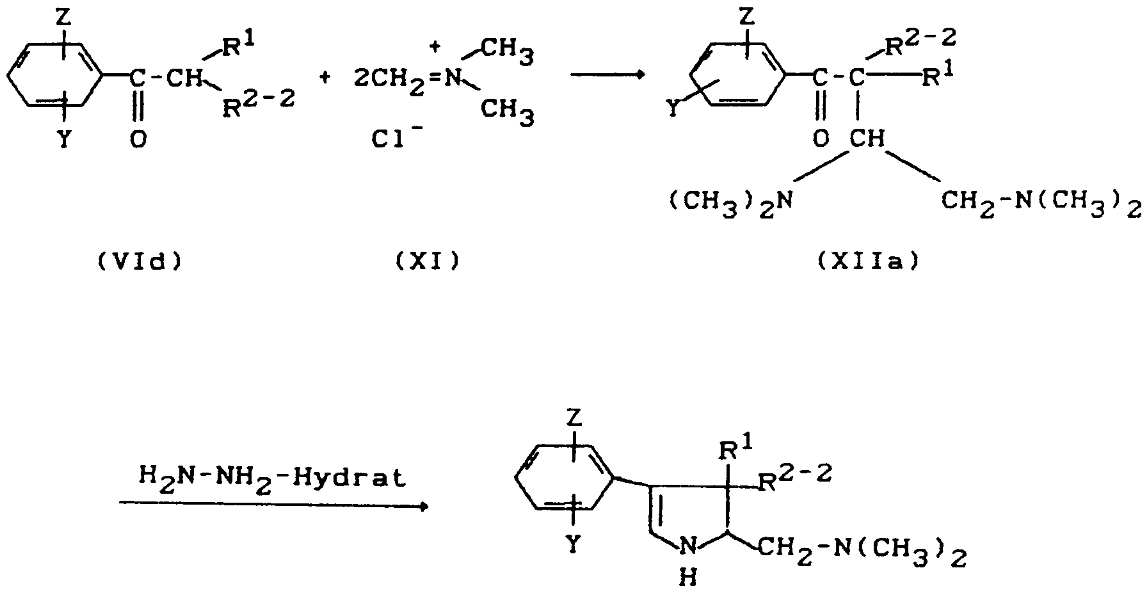


(VId) (XI) (XIIa)

(II)

Die Verbindungen der Formel (VIa) und (VId) sind teilweise neu. Sie werden durch Umsetzung der Verbindungen

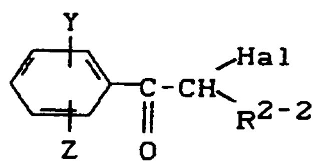

(X)

worin

- $R^{2-2}$ für Wasserstoff, Alkyl oder Alkoxycarbonyl steht,
- X und Y die oben angegebene Bedeutung haben und
- Hal für Halogen, insbesondere Brom steht,

mit der Verbindung $R^1$-H unter Zuhilfenahme einer organischen oder anorganischer Base unter Halogenwasserstoffeliminierung hergestellt. Bei den Verbindungen der Formel (X) und $R^1$-H handelt es sich um bekannte Stoffe (vgl. z.B. Comprehensive Heterocyclic Chemistry, A.R. Katritzky, Vol. 5, Part 4A, Pergamon Press und US 3 780 052).

Bei der Durchführung des erfindungsgemäßen Verfahrens gemäß der Verfahrensvariante (B) werden Verbindungen der Formel (IV)

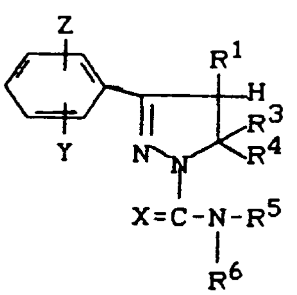

(IV)

in welcher X, Y, Z, $R^1$, $R^3$, $R^4$, $R^5$ und $R^6$ die oben angegebene Bedeutung besitzen mit einer starken Base, vorzugsweise einer metallorganischen Verbindung, insbesondere mit Butyl-Lithium im inerten organischen

Lösungsmittel bei Temperaturen von -50 bis 0 ° C, insbesondere von -30 ° C bis -15 ° C, gegebenenfalls in Anwesenheit einer Schutzgasatmosphäre, insbesondere Edelgasatmosphäre wie z.B. Argon, umgesetzt und anschließend mit einem Halogenid Hal-$R^2$

worin

$R^{2-1}$ für Alkyl, gegebenenfalls substituiertes Cycloalkyl, Halogenalkyl, Alkoxycarbonyl oder Halogenalkylthio steht,

bei 0 bis 60 ° C, insbesondere bei -10 bis -40 ° C umgesetzt und durch Zusatz von Wasser und Extraktion mit Ether in üblicher Weise aufgearbeitet.

Man erhält dann Verbindungen der Formel (XIII)

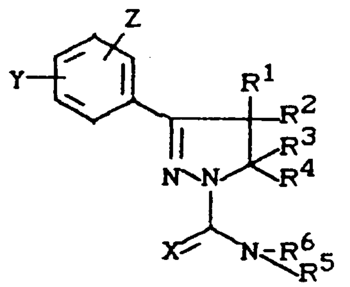

(XI)

wobei X, Y, Z, $R^1$, $R^2$, $R^3$, $R^4$, $R^5$, $R^6$ und $R^9$ die oben angegebenen Bedeutungen besitzen.

Die Wirkstoffe eignen sich zur Bekämpfung von tierischen Schädlingen, vorzugsweise Arthropoden und Nematoden, insbesondere Insekten und Spinnentieren, die in der Landwirtschaft, in der Tierhaltung, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus,

Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Hydrotaea spp., Haematobia spp., Glossina spp., Melophagus spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp., Ctenocephalides spp.. Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Ornithonyssus spp., Eriophyes ribis, Phyllocoptruta oleivora, Boophilus spp., Rhipicephalus spp., Amblyomma spp., Hyalomma spp., Ixodes spp., Dermacentor spp., Haemaphysalis spp., Otobius spp., Psoroptes spp., Chorioptes spp., Sarcoptes spp., Psorergates spp., Demodex spp., Notoedres spp., Tarsonemus spp., Bryobia praetiosa, Panonychus spp., Tetranychus spp..

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten und Endoparasiten) wie Schildzecken, Lederzecken, Räudemilben, Laufmilben, Fliegen (stechend und leckend), parasitierende Fliegenlarven, Läuse, Haarlinge, Federlinge, Flöhe und endoparasitisch lebende Würmer.

Sie sind gegen normalsensible und resistente Arten und Stämme sowie gegen alle parasitierenden und nicht parasitierenden Entwicklungsstadien der Ekto- und Endoparasiten wirksam.

Die erfindungsgemäßen Wirkstoffe zeichnen sich durch eine hohe insektizide und akarizide Wirksamkeit aus.

Sie lassen sich mit besonders gutem Erfolg zur Bekämpfung von pflanzenschädigenden Insekten, wie beispielsweise gegen die Larven des Meerettichblattkäfers (Phaedon cochleariae) oder gegen die Raupen der Kohlschabe (Plutella maculipennis) oder gegen die Tabakknospenraupe (Heliothis virescens); einsetzen.

Darüberhinaus lassen sie sich mit besonders gutem Erfolg zur Bekämpfung von parasitisch lebenden Warmblüterschädlingen, wie beispielsweise gegen die Larven der Goldfliege (Lucilia cuprina), gegen Musca domestica und gegen Periplaneta americana einsetzen.

Die Wirkstoffe können in die übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte ali phatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem

Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Insekten, Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemäßen Wirkstoffe geschieht auf diesem Gebiet in bekannter Weise, wie durch äußerliche Anwendung in Form beispielsweise des Tauchens (Dippen), Sprühens (Sprayen), Aufgießen (pour-on and spot-on) und des Einpuderns sowie durch parenterale Anwendung in Form beispielsweise der Injektion sowie ferner durch das "feed-through"-Verfahren. Daneben ist auch eine Anwendung als Formkörper (Halsband, Ohrmarke), sowie eine Anwendung in Form der sogenannten Umgebungsbehandlung möglich.

Die nachfolgenden Beispiele beschreiben die Herstellung und die Verwendung erfindungsgemäßer Wirkstoffe ohne sie darauf zu beschränken.

Herstellungsbeispiele

Beispiel 1

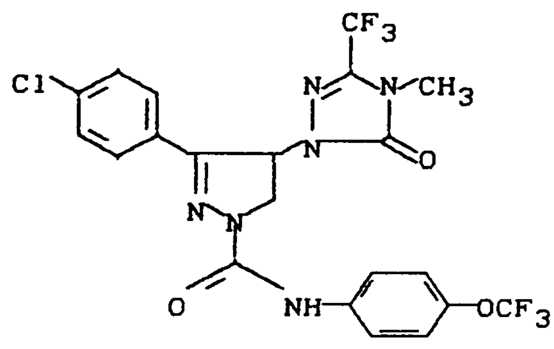

2,76 g (0,008 Mol) 3-(4'-Chlorphenyl)-4-(4''-Methyl-3''-trifluormethyl-$\Delta^{2''}$-1'',2'',4''-triazolin-5''-on-1''-yl)-4,5-dihydropyrazol werden in 20 ml Acetonitril (wasserfrei) bei 60 °C klar gelöst mit 1,7 g 4-Trifluormethoxy-phenylisocyanat versetzt und 2 Tropfen Triäthylamin zugegeben. Anschließend läßt man 2 Stunden bei Raumtemperatur stehen, Das Lösungsmittel wird anschließend im Vakuum abgezogen, der Rückstand mit 20 ml Diethyläther versetzt und nach 2 Stunden der entstandene Niederschlag abgesaugt. Man erhält 2,0 g (45,6 % der Theorie) 3-(4'-Chlorphenyl)-4-(4''-Methyl-3''-trifluormethyl-$\Delta^{2''}$-1'',2'',4''-triazolin-5''-on-1''-yl)-4,5-dihydro-1-pyrazol-carbonsäure-(4-trifluormethoxy)-anilid mit einem Schmelzpunkt von 241 °C.

In analoger Weise zu Beispiel 1 und unter Berücksichtigung der Angaben in der Beschreibung zu den erfindungsgemäßen Verfahren werden die nachfolgend in Tabelle 2 aufgeführten Endprodukte der Formel (I) erhalten:

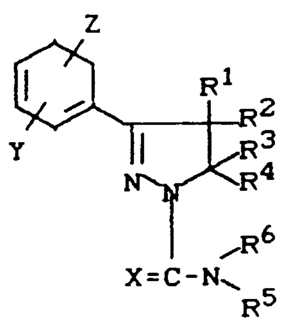

(I)

Tabelle 2

| Bsp.Nr. | $R^1$ | $R^2$ | $R^3$ | $R^4$ | $R^5$ | $R^6$ | Y | Z | X | Schmelz-punkt °C |
|---|---|---|---|---|---|---|---|---|---|---|
| 2 | 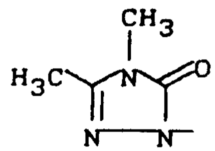 | H | H | H | H | 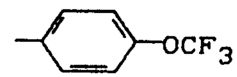 | 4Cl | H | O | 211 |
| 4 | 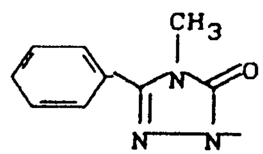 | H | H | H | H | 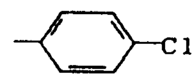 | 4Cl | H | O | 241 |
| 3 | 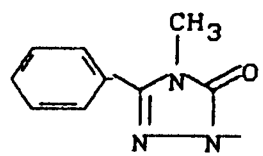 | H | H | H | H | 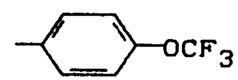 | 4Cl | H | O | 218 |
| 5 | 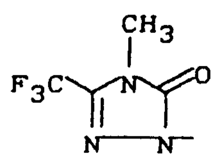 | H | H | H | H | 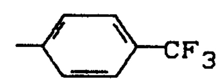 | 4Cl | H | O | 230 |

Tabelle 2 - Fortsetzung

| Bsp.Nr. | $R^1$ | $R^2$ | $R^3$ | $R^4$ | $R^5$ | $R^6$ | Y | Z | X | Schmelzpunkt °C |
|---|---|---|---|---|---|---|---|---|---|---|
| 6 | 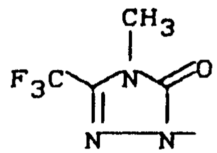 | H | H | H | H | 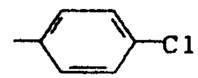 | 4Cl | H | O | 256 |
| 7 | 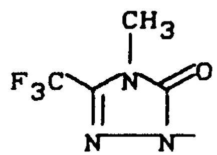 | H | H | H | H | 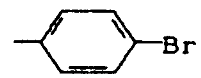 | 4Cl | H | O | 257 |
| 8 | dito | H | H | H | H | 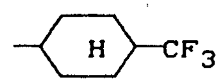 | 4Cl | H | O | 192 |
| 9 | dito | H | H | H | H | 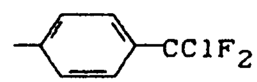 | 4Cl | H | O | 226 |
| 10 | dito | H | H | H | H | 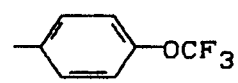 | H | H | O | 224 |

Tabelle 2 - Fortsetzung

| Bsp.Nr. | $R^1$ | $R^2$ | $R^3$ | $R^4$ | $R^5$ | $R^6$ | Y | Z | X | Schmelzpunkt °C |
|---|---|---|---|---|---|---|---|---|---|---|
| 11 | dito | H | H | H | H | 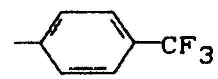 | H | H | O | 238 |
| 12 | dito | H | H | H | H | 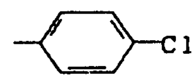 | H | H | O | 236 |
| 13 | dito | H | H | H | H | 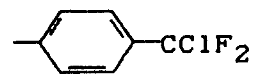 | H | H | O | 204 |
| 14 | dito | H | H | H | H | 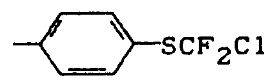 | H | H | O | 221 |
| 15 | dito | H | H | H | H | 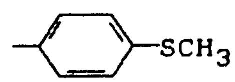 | H | H | O | 203 |

Tabelle 2 - Fortsetzung

| Bsp.Nr. | $R^1$ | $R^2$ | $R^3$ | $R^4$ | $R^5$ | $R^6$ | Y | Z | X | Schmelzpunkt °C |
|---|---|---|---|---|---|---|---|---|---|---|
| 16 | dito | H | H | H | H | 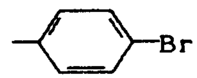 | H | H | O | 241 |
| 17 | 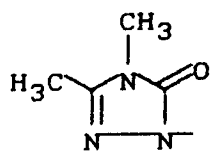 | H | $-CH_2$<br>\|<br>$N(CH_3)_2$ | H | H | 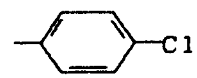 | 4Cl | H | O | 229 |
| 18 | 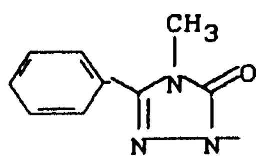 | H | H | H | H | 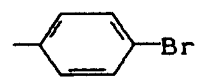 | 4Cl | H | O | 234 |
| 19 | 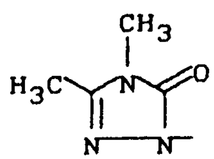 | H | $-CH_2$<br>\|<br>$N(CH_3)_2$ | H | H | 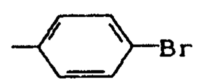 | 4Cl | H | O | 242 |

Tabelle 2 - Fortsetzung

| Bsp.Nr. | $R^1$ | $R^2$ | $R^3$ | $R^4$ | $R^5$ | $R^6$ | Y | Z | X | Schmelzpunkt °C |
|---|---|---|---|---|---|---|---|---|---|---|
| 20 | 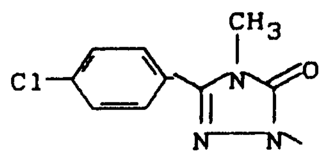 | H | H | H | H | 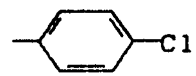 | 4Cl | H | O | |
| 21 | 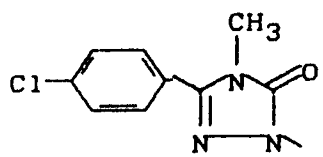 | H | H | H | H | 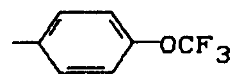 | 4Cl | H | O | |
| 22 | dito | H | H | H | H | 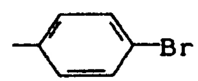 | 4Cl | H | O | |

Herstellung der Ausgangsverbindungen

Beispiel (II-1)

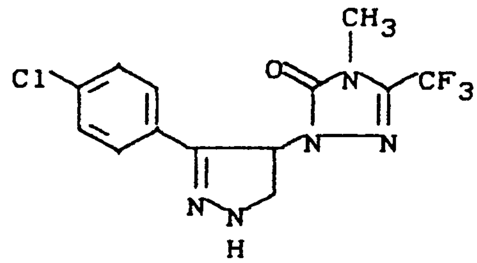

11,2 g (0,035 Mol) α-(4-Methyl-3-trifluormethyl-$\Delta^2$-1,2,4-triazolin-5-on-1-yl)-4'-chloraceto-phenon werden in 100 ml absolutem Acetonitril klar gelöst, 10 g (0,107 Mol) N,N-Dimethylmethylenimmoniumchlorid zugegeben und 4 Stunden zum Rückfluß erhitzt, bis nach DC-Kontrolle kein Ausgangsmaterial mehr sichtbar ist. Bei 70 °C werden dann 20 ml Hydrazinhydrat zugegeben und 10 Minuten nachgerührt. Anschließend wird im Vakuum eingeengt, der Rückstand mit 200 ml Wasser versetzt und mit Essigester extrahiert. Die organische Phase wird abgetrennt über $MgSO_4$ getrocknet und eingeengt. Man erhält 12 g (99 % der Theorie) 3-(4'-Chlorphenyl)-4-(4''-methyl-3''-trifluormethyl-$\Delta^{2''}$-1'',2'',4''-triazolin-5''-on-1''-yl)-4,5-dihydropyrazol als hellgelben Feststoff mit dem Schmelzpunkt 198 °C.

In analoger Weise zu Beispiel (II-1) und unter Berücksichtigung der Angaben in der Beschreibung zu den erfindungsgemäßen Verfahren werden die nachfolgend in Tabelle 3 aufgeführten Ausgangsverbindungen der Formel (II) erhalten:

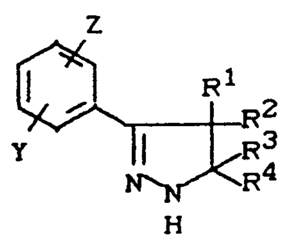

(II)

Tabelle 3

| Bsp.Nr. | $R^1$ | $R^2$ | $R^3$ | $R^4$ | Y | Z | physik. Konst. |
|---|---|---|---|---|---|---|---|
| II-2 | 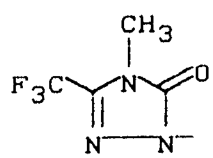 | H | H | H | H | H | $^1$H-NMR*: ($\delta$=7,3-7,75(5H,m); 6,05(1H,q);3,71-3,95(2H,m);3,36 (3H,s) |
| II-3 | 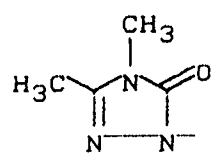 | H | H | H | 4Cl | H | Öl |
| II-4 | dito | H | $CH_2$-$N(CH_3)_2$ | H | 4Cl | H | $^1$H-NMR**: ($\delta$=5,63(1H,d);3,95 (1H,m);2,20-2,45 (2H,m);2,2(6H,s) 3,1(3H,s);2,1(3H,s) |

* Die $^{1}H$-NMR-Spektren wurden in Deuterochloroform ($CDCl_3$) mit Tetramethylsilan (TMS) als innerem Standard aufgenommen. Angegeben ist die chemische Verschiebung als $\delta$-Wert in ppm.

** Die $^{1}H$-NMR-Spektren wurden deuteriertem Dimethylsulfoxid $(CD_3)_2SO$ mit Tetramethylsilan (TMS) als innerem Standard aufgenommen. Angegeben ist die chemische Verschiebung als $\delta$-Wert in ppm.

Tabelle 3

| Bsp.Nr. | $R^1$ | $R^2$ | $R^3$ | $R^4$ | Y | Z | physik. Konst. |
|---|---|---|---|---|---|---|---|
| II-5 | 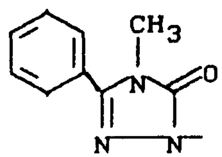 | H | H | H | 4Cl | H | Öl |
| II-6 | 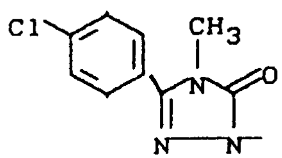 | H | H | H | 4Cl | H | Öl |

Herstellung der Vorprodukte

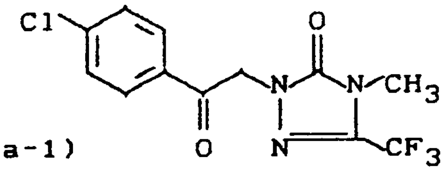

(VIa-1)

1,8 g (0,075 Mol) Natriumhydrid werden in 100 ml DMF (wasserfrei) unter Schutzgasatmosphäre suspendiert und zu dieser Suspension 10 g (0,06 Mol) 4-Methyl-3-trifluormethyl-$\Delta^2$-1,2,4-triazolin-5-on gelöst in 100 ml DMF (wasserfrei) bei 20 °C zugetropft. Anschließend wird 3 Stunden bei 60 °C nachgerührt bis eine klare Lösung entstanden ist. Hierzu werden 14 g (0,06 Mol) 4-Chlorphenacylbromid gelöst in 100 ml DMF (wasserfrei) bei 20 bis 30 °C zugetropft und dann noch 1 Stunde bei Raumtemperatur nachgerührt. Die Reaktionsmischung wird mit 2 l Wasser versetzt, der ausfallende Feststoff abgesaugt und anschließend nochmals mit Petroläther suspendiert. Nach dem Absaugen erhält man 11,5 g (52 % der Theorie) α-(4-Methyl-3-trifluormethyl-$\Delta^2$-1,2,4-triazolin-5-on-1-yl)-4'-chloracetophenon als hellgelben Feststoff.

$^1$H-NMR*(δ = 7,48-7,95 (4H,m) 5,37 (2H,s), 3,43 (3H,s).

In analoger Weise zu Beispiel (VIa-1) und unter Berücksichtigung der Angaben in der Beschreibung zu den erfindungsgemäßen Verfahren werden die nachfolgend in Tabelle 4 aufgeführten Vorprodukte der Formel (VIa) erhalten:

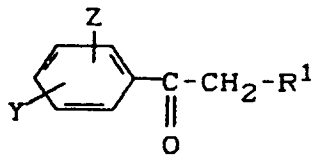

(VIa)

* Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Tabelle 4

| Bsp.Nr. | $R^1$ | Y | Z | physikal. Konst. |
|---|---|---|---|---|
| (VIa-2) | 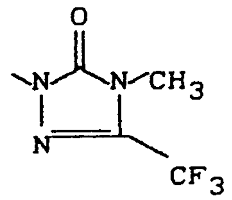 | H | H | Öl |
| (VIa-3) | 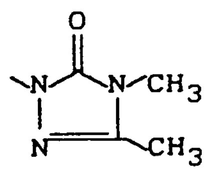 | 4Cl | H | 148° C |
| (VIa-4) | 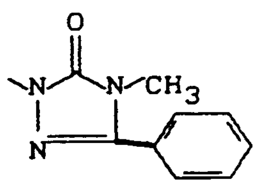 | 4Cl | H | 128° C |
| (VIa-5) | 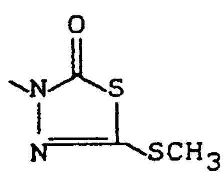 | 4Cl | H | 126° C |
| (VIa-6) | dito | 4Br | H | 141° C |
| (VIa-7) | 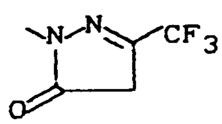 | 4Cl | H | 112° C |

Tabelle 4 - Fortsetzung

| Bsp.Nr. | $R^1$ | Y | Z | physikal. Konst. |
|---|---|---|---|---|
| (VIa-8) | 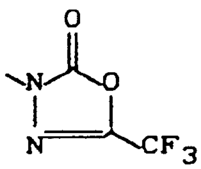 | 4Cl | H | Öl |
| (VIa-11) | 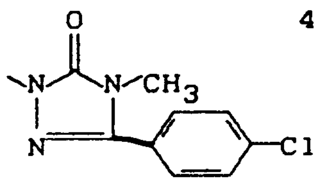 | 4Cl | H | |
| (VIa-9) | 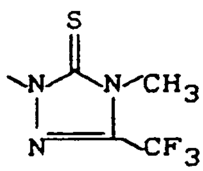 | 4Cl | H | |
| (VIa-10) | 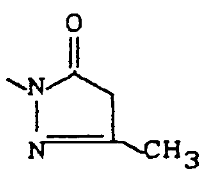 | 4Cl | H | |

Anwendungsbeispiele

In den folgenden Anwendungsbeispielen wurden die nachstehend aufgeführten Verbindungen als Vergleichssubstanzen eingesetzt.

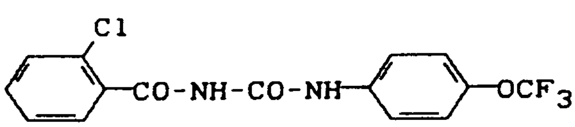 (A)

Triflumuron = 2-Chlor-N[[4-(trifluormethoxy)-phenyl]-amino]-carbonyl]-benzamid
(bekannt aus: DE-A 2 601 780)

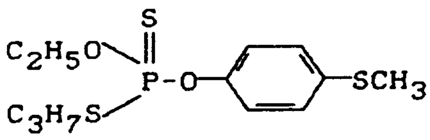

(B)

Sulprofos = O-(Ethyl-O-(4-methylthio)-phenyl)-S-propyl-dithiophosphat (bekannt aus: DE-A 2 111 414)

Beispiel A

Phaedon-Larven-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik: 1, 5, 6, 7, 8, 10, 11, 12, 13, 14 und 16.

Beispiel B

Heliothis virescens - Test

Lösungsmittel : 7 Gewichtsteile Dimethylformamid
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) werden durch Tauchen in die Wirkstoffzubereitung der gewünschten Konzentration behandelt und mit der Tabakknospenraupe (Heliothis virescens) besetzt, solange die Blätter noch feucht sind.

Nach der gewünschten Zeit wird die Abtötung in % bestimmt. Dabei bedeutet 100%, daß alle Raupen abgetötet wurden; 0% bedeutet, daß keine Raupen abgetötet wurden.

Bei diesem Test zeigen z.B. die folgenden Verbindungen der Herstellungsbeispiele überlegene Wirksamkeit gegenüber dem Stand der Technik: 1, 5 und 9.

Beispiel C

Plutella- Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik: 1, 10 und 11

Beispiel D

Blowfly-Larven-Test

Testtiere: Lucilia cuprina-Larven
Emulgator: 35 Gew.-Teile Ethylenglykolmonomethylether
35 Gew.-Teile Nonylphenolglykolether

Zur Herstellung einer zweckmäßigen Wirkstoffzubereitung vermischt man drei Gewichtsteile Wirkstoff mit sieben Gewichtsteilen des oben angegebenen Gemisches und verdünnt das so erhaltene Emulsionskonzentrat mit Wasser auf die jeweils gewünschte Konzentration.

Etwa 20 Lucilia cuprina res.-Larven werden in ein Teströhrchen gebracht, welches ca. 1 $cm^3$ Pferdefleisch und 0,5 ml der Wirkstoffzubereitung enthält. Nach 24 Stunden wird die Wirksamkeit der Wirkstoffzubereitung ermittelt. Dabei bedeutet 100 %, daß alle Blowfly-Larven abgetötet wurden; 0 % bedeutet, daß keine Blowfly-Larven abgetötet wurden.

In diesem Test zeigten z.B. die folgenden Verbindungen der Herstellungsbeispiele überlegene Wirkung gegenüber dem Stand der Technik: 1, 5, 8, 9, 10, 11, 12, 13, 14 und 16.

Beispiel: E

$LT_{100}$-Test

Testtiere: Musca domestica, Stamm WHO-N
Lösungsmittel:
35 Gewichtsteile Ethylenglykolmonomethylether
35 Gewichtsteile Nonylphenolpolyglykolether

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

2 ml dieser Wirkstoffzubereitung werden auf Filterpapierschalen (Ø 9,5 cm) pipettiert, die sich in Petrieschalen entsprechender Größe befinden. Nach Trocknung der Filterscheiben werden 25 Testtiere in die Petrieschale überführt und abgedeckt.

Der Zustand der Testtiere wird bis zu 6 Stunden laufend kontrolliert. Es wird diejenige Zeit ermittelt, die für eine 100 %ige knock down-Wirkung erforderlich ist. Wird die $LT_{100}$ nach 6 Stunden nicht erreicht, wird der %-Satz der knock down gegangenen Testtiere festgestellt.

In diesem Test zeigte z.B. die Verbindung des Herstellungsbeispiels 8 bei einer beispielhaften Konzentration von 1000 ppm a.i. eine $LT_{100}$ von 100 Minuten.

**Patentansprüche**

1. Substituierte Carbamoylpyrazoline der allgemeinen Formel (I)

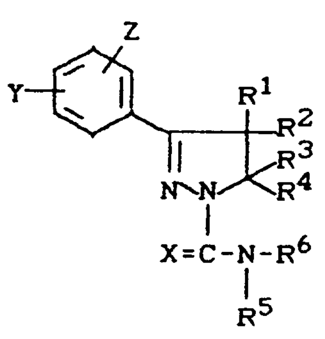

(I)

in welcher

$R^1$ für einen jeweils gegebenenfalls substituierten und über Stickstoff gebundenen Azolinon-, Azolinthion-oder Azoliniminorest steht,

$R^2$ für Wasserstoff, Alkyl, gegebenenfalls substituiertes Cycloalkyl, Halogenalkyl, Halogenalkylthio oder Alkoxycarbonyl steht,

$R^3$ für Wasserstoff, Alkyl oder für eine Gruppierung

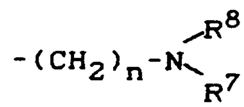

oder $-(CH_2)_n-O-R^9$ steht,
worin

$R^7$ und $R^8$ jeweils unabhängig voneinander für Wasserstoff, Alkyl oder Aryl stehen und

$R^9$ für Wasserstoff, Alkyl oder Aryl steht und

n für die Zahlen 1 bis 6 steht,

$R^4$ für Wasserstoff oder Alkyl steht,

$R^5$ für Wasserstoff, Alkyl, Phenyl oder Alkylthio steht,

$R^6$ für gegebenenfalls substituiertes Alkyl, gegebenenfalls substituiertes Cycloalkyl oder für den Rest

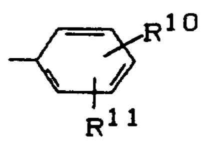

steht,
worin $R^{10}$ und $R^{11}$ gleich oder verschieden sein können und für Wasserstoff, Halogen, Alkyl, Nitro, Cyano, Halogenalkyl, Alkoxy, Halogenalkoxy, Alkylthio, Halogenalkylthio, gegebenenfalls substituiertes Phenoxy, gegebenenfalls substituiertes Mono-oder Dialkylamino, gegebenenfalls substituiertes Cycloalkyl, Alkoxycarbonyl, gegebenenfalls substituiertes Arylthio, Alkenyloxy, Alkinyl, Alkylthionyl, Alkylsulfonyl, Halogenalkylthionyl, Halogenalkylsulfonyl, Halogenalkoxycarbonyl stehen oder wobei $R^{10}$ und $R^{11}$ zusammen für einen bivalenten gegebenenfalls ein oder zwei Sauerstoffatome enthaltenden und gegebenenfalls substituierten Rest stehen,

X für Sauerstoff oder Schwefel steht und

Y und Z gleich oder verschieden sein können und für Wasserstoff, Alkyl, Halogen, Halogenalkyl, Alkoxy, Alkylthio, Halogenalkoxy, Halogenalkylthio, Alkoxycarbonyl, Halogenalkoxycarbonyl, gegebenenfalls substituiertes Aryloxy, gegbenenfalls substituiertes Arylthio, Alkenyloxy, Alkinyl, Alkylthionyl, Alkylsulfonyl, Halogenalkylthionyl, Halogenalkylsulfonyl, Nitro oder Cyano stehen, oder wobei Y und Z zusammen für gegebenenfalls durch Halogen substituiertes 3,4-Methylendioxy oder 3,4-Ethylendioxy stehen.

**2.** Verfahren zur Herstellung von substituierten Carbamoylpyrazolinen der allgemeinen Formel (I)

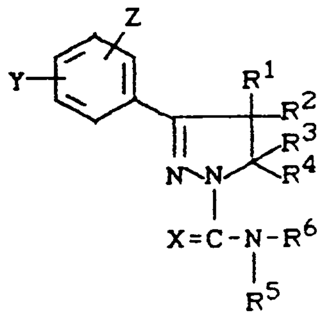

(I)

in welcher

$R^1$ für einen jeweils gegebenenfalls substituierten Azolinon-, Azolinthion-oder Azoliniminorest steht,

$R^2$ für Wasserstoff, Alkyl, gegebenenfalls substituiertes Cycloalkyl, Halogenalkyl, Halogenalkylthio oder Alkoxycarbonyl steht,

$R^3$ für Wasserstoff, Alkyl oder für eine Gruppierung

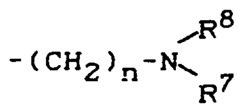

oder $-(CH_2)_n-O-R^9$ steht,
worin

$R^7$ und $R^8$ jeweils unabhängig voneinander für Wasserstoff, Alkyl oder Aryl stehen und

$R^9$ für Wasserstoff, Alkyl oder Aryl steht und

n für die Zahlen 1 bis 6 steht,

$R^4$ für Wasserstoff oder Alkyl steht,

$R^5$ für Wasserstoff, Alkyl, Phenyl oder Alkylthio steht,

$R^6$ für gegebenenfalls substituiertes Alkyl, gegebenenfalls substituiertes Cycloalkyl oder für den Rest

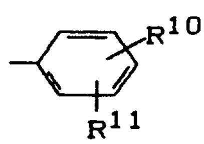

steht,
worin $R^{10}$ und $R^{11}$ gleich oder verschieden sein können und für Wasserstoff, Halogen, Alkyl, Nitro, Cyano, Halogenalkyl, Alkoxy, Halogenalkoxy, Alkylthio, Halogenalkylthio, gegebenenfalls substituiertes Phenoxy, gegebenenfalls substituiertes Mono- oder Dialkylamino, gegebenenfalls substituiertes Cycloalkyl, Alkoxycarbonyl, gegebenenfalls substituiertes Arylthio, Alkenyloxy, Alkinyl, Alkylthionyl, Alkylsulfonyl, Halogenalkylthionyl, Halogenalkylsulfonyl, Halogenalkoxycarbonyl stehen oder wobei $R^{10}$ und $R^{11}$ zusammen für einen bivalenten gegebenenfalls ein oder zwei Sauerstoffatome enthaltenden und gegebenenfalls substituierten Rest stehen,

X für Sauerstoff oder Schwefel steht und

Y und Z gleich oder verschieden sein können und für Wasserstoff, Alkyl, Halogen, Halogenalkyl, Alkoxy, Alkylthio, Halogenalkoxy, Halogenalkylthio, Alkoxycarbonyl, Halogenalkoxycarbonyl, gegebenenfalls substituiertes Aryloxy, gegbenenfalls substituiertes Arylthio, Alkenyloxy, Alkinyl, Alkylthionyl, Alkylsulfonyl, Halogenalkylthionyl, Halogenalkylsulfonyl, Nitro oder Cyano stehen, oder wobei Y und Z zusammen für gegebenenfalls durch Halogen substituiertes 3,4-Methylendioxy oder 3,4-Ethylendioxy stehen,

dadurch gekennzeichnet, daß man

A) zum Erhalt von Carbamoylpyrazolinen der Formel (I), in welcher $R^5$ für Wasserstoff steht, Pyrazolinderivate der Formel (II)

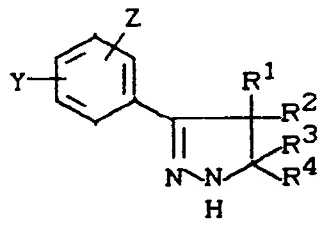

(II)

in welcher Y, Z, $R^1$, $R^2$, $R^3$ und $R^4$ die oben angegebene Bedeutung besitzen, mit Isocyanaten bzw. Isothiocyanaten der Formel (III)

$X = C = N\text{-}R^6$ (III)

in welcher X und $R^6$ die oben angegebene Bedeutung haben, gegebenenfalls in Gegenwart von Basen umsetzt oder daß man

B) zum Erhalt von Carbamoylpyrazolinen der Formel (I), in welcher $R^2$ für Alkyl, Cycloalkyl, Halogenalkyl, Halogenalkylthio oder Alkoxycarbonyl steht, Verbindungen der Formel (IV)

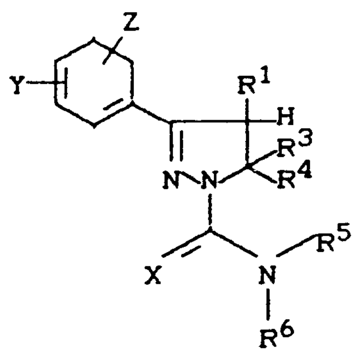

(IV)

in welcher

x, Y, Z, $R^1$, $R^3$, $R^4$, $R^5$ und $R^6$ die oben angebenene Bedeutung haben,

mit Verbindungen der Formel (V)

$Hal\text{-}R^{2-1}$ (V)

in welcher

Hal für Halogen steht und

$R^{2-1}$ für Alkyl, gegebenenfalls substituiertes Cycloalkyl, Halogenalkyl, Halogenalkylthio oder Alkoxycarbonyl steht,

in wasserfreiem Medium unter Zusatz einer starken Base umsetzt.

3. Schädlingsbekämpfungsmittel, gekennzeichnet durch einen Gehalt an mindestens einem substituierten Carbamoylpyrazolin der Formel (I) gemäß Anspruch 1.

4. Verwendung von substituierten Carbamoylpyrazolinen der Formel (I) gemaß Anspruch 1 zur Bekämpfung von Schädlingen.

5. Verfahren zur Bekämpfung von Schädlingen, dadurch gekennzeichnet, daß man Carbamoylpyrazoline der Formel (I) gemäß Anspruch 1 auf Schädlinge und/oder ihren Lebensraum einwirken läßt.

6. Verfahren zur Herstellung von Schädlingsbekämpfungsmitteln, dadurch gekennzeichnet, daß man Carbamoylpyrazolin der Formel (I) gemäß Anspruch 1 mit Streckmitteln und/oder oberflächenaktiven Mitteln vermischt.

7. Verwendung von Carbamoylpyrazoline der Formel (I) gemäß Anspruch 1 zur Herstellung von Schädlingsbekämpfungsmitteln.

Europäisches Patentamt

# EUROPÄISCHER RECHERCHENBERICHT

Nummer der Anmeldung
EP 93 11 5420

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| D,X | EP-A-0 466 408 (ROHM AND HAAS COMPANY) 15. Januar 1992<br>* Ansprüche 1,22 *<br>--- | 1,3 | C07D403/04<br>C07D417/04<br>C07D413/04<br>C07D231/22<br>A01N47/38 |
| P,A | EP-A-0 529 451 (BAYER AG) 3. März 1993<br>* Anspruch 3 *<br>----- | 1,3 | |
| | | | RECHERCHIERTE SACHGEBIETE (Int.Cl.5) |
| | | | C07D<br>A01N |

Der vorliegende Recherchenbericht wurde für alle Patentansprüche erstellt

| Recherchenort | Abschlußdatum der Recherche | Prüfer |
|---|---|---|
| DEN HAAG | 14. Januar 1994 | VOYIAZOGLOU, D |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)